# EUROPEAN PATENT SPECIFICATION

(11) **EP 4 395 774 B1**
(45) Date of publication and mention of the grant of the patent: **20.08.2025**
(21) Application number: 22768985.8
(22) Date of filing: 30.08.2022
(51) Int. Cl.: A61K 31/4184, A61K 31/496, A61K 31/5025, A61K 31/506, A61K 31/517, A61K 31/519, A61K 45/06, A61P 35/00

(54) **COMBINATION THERAPY WITH A MEK INHIBITOR AND A SRC INHIBITOR FOR THE TREATMENT OF COLORECTAL CANCER**
KOMBINATIONSTHERAPIE MIT EINEM MEK-INHIBITOR UND EINEM SRC-INHIBITOR ZUR BEHANDLUNG VON KOLOREKTALKREBS
THÉRAPIE COMBINÉE COMPRENANT UN INHIBITEUR MEK ET UN INHIBITEUR SRC POUR LE TRAITEMENT DU CANCER COLORECTAL

(30) Priority: 30.08.2021 US 202163238244 P
(43) Date of publication of application: 10.07.2024
(73) Proprietor: Yeda Research and Development Co. Ltd, 7610002 Rehovot (IL); The Medical Research, Infrastructure and Health Services Fund of the Tel Aviv Medical Center, 6423906 Tel Aviv (IL)
(72) Inventor: STRAUSSMAN, Ravid, 7685437 Mazkeret Batia (IL); GAVERT, Nancy, 7610002 Rehovot (IL); ZWANG, Yaara, 7610002 Rehovot (IL)
(74) Representative: Stolmár & Partner Patentanwälte PartG mbB
(86) International application number: PCT/IL2022/050947
(87) International publication number: WO 2023/031925

(56) References cited:
- WO-A2-2012/166824
- US-A1- 2021 154 198
- PARK SANG-MIN ET AL: "Feedback analysis identifies a combination target for overcoming adaptive resistance to targeted cancer therapy", ONCOGENE, NATURE PUBLISHING GROUP UK, LONDON, vol. 39, no. 19, 10 March 2020 (2020-03-10), pages 3803 - 3820, XP037112817, ISSN: 0950-9232, [retrieved on 20200310], DOI: 10.1038/S41388-020-1255-Y
- SKOPELITOU DIAMANTO ET AL: "A Novel Low-Risk Germline Variant in the SH2 Domain of the SRC Gene Affects Multiple Pathways in Familial Colorectal Cancer", JOURNAL OF PERSONALIZED MEDICINE, vol. 11, no. 4, 1 April 2021 (2021-04-01), pages 262, XP093001806, DOI: 10.3390/jpm11040262
- ZHU S ET AL: "Decreased CHK protein levels are associated with Src activation in colon cancer cells", ONCOGENE, NATURE PUBLISHING GROUP UK, LONDON, vol. 27, no. 14, 15 October 2007 (2007-10-15), pages 2027 - 2034, XP037742857, ISSN: 0950-9232, [retrieved on 20071015], DOI: 10.1038/SJ.ONC.1210838

## Description

### RELATED APPLICATION

This application claims the benefit of priority of US Application No. 63/238,244 filed 30 August, 2021, the contents of which are incorporated herein by reference in their entirety.

### FIELD AND BACKGROUND OF THE INVENTION

The present invention, in some embodiments thereof, relates to treatment of colorectal cancer.

The tumor microenvironment is an essential factor modulating the response of cancer cells to anti-cancer therapy. Multiple stromal components, such as immune cells, exosomes, fibroblasts, adipose cells, and even bacteria can affect tumor cell response to drug treatment. The complexity of the tumor tissue may account for the difficulties in using *in vitro* models for preclinical studies, where tumor architecture and complexity are not recapitulated. Even *in vivo* mouse models are often insufficient for predicting the response of cancer patients to treatment, therefore many drugs shown to be effective in preclinical mice models have eventually failed in clinical trials. Indeed, it has been estimated that less than 10% of oncology treatments that demonstrate efficacy in pre-clinical models translate to better patient care.

CRC treatment options are limited for locally advanced, metastatic and recurrent disease. For the vast majority of CRC patients, therapeutic regimes have not changed dramatically in the past decade, nor has the prognosis improved markedly, even after the introduction of targeted immunotherapies.

*Ex vivo* organ culture (EVOC) was developed more than a century ago to enable the culturing of fresh tissues outside the body (13), and has since been used for numerous applications, including oncology-related studies (14), although many involved only short culture times of 24-48 hours (15,16). EVOC preserves the tumor microenvironment, architecture and genetic heterogeneity (13)

### Additional background art includes:

WO2018/185760;
WO2019/145964;
US 2021/154198 A1

Park Sang-Min et al. Feedback analysis identifies a combination target for overcoming adaptive resistance to targeted cancer therapy, Oncogene, Nature Publishing Group UK, LONDON, 39 (19): 3803-3820 (2020).

### SUMMARY OF THE INVENTION

According to one aspect of the present invention, there is disclosed a method of treating colorectal cancer (CRC), the method comprising administering to a subject in need thereof a therapeutically effective amount of a Mitogen/extracellular signal-regulated kinase (MEK) inhibitor and a SRC Proto-Oncogene, Non-Receptor Tyrosine Kinase (SRC) inhibitor, wherein cancer cells of the subject express an amount of SRC p419 above a predetermined level and do not express a KRAS G12 mutation, thereby treating the CRC.

According to an aspect of the present invention, there is provided a combination comprising a therapeutically effective amount of a MEK inhibitor and a SRC inhibitor for use in treating colorectal cancer (CRC), wherein cells of the CRC express an amount of SRC p419 above a predetermined level and do not express a KRAS G12 mutation.

According to an aspect of the present invention, there is provided a method of selecting treatment to colorectal cancer (CRC) for a subject in need thereof, the method comprising determining a level of SRC p419 and a RAS mutation to exclude a KRAS G12 mutation in cancer cells of the subject, wherein a SRC p419 level above a predetermined level and an absence of a KRAS G12 mutation, is indicative of an efficacious treatment with of a MEK inhibitor and a SRC inhibitor.

According to embodiments of the invention, the method further comprises testing sensitivity to the treatment in an *ex-vivo* organ culture (EVOC) model.

According to embodiments of the invention, the method comprises any of:
(i) testing sensitivity to the MEK inhibitor and the SRC inhibitor in the EVOK model by monitoring cell killing;
(ii) testing sensitivity to the MEK inhibitor and the SRC inhibitor in cells of the EVOK model by determining a level of MEK phosphorylation (pMEK), wherein a level below a predetermined or control is indicative of an efficacious treatment; and/or
(iii) testing sensitivity to the SRC inhibitor in cells of the EVOK model by determining level ERK1/2 phosphorylation (pERK1/2), wherein a level below a predetermined or control is indicative of an efficacious treatment.

According to embodiments of the invention, the EVOC model comprises culturing in a culture medium a precision-cut tissue slice of the CRC placed on a tissue culture insert, wherein the precision-cut tissue slice is maintained in a highly oxygenated atmosphere containing about 80 % oxygen and wherein the culture is rotationally agitated facilitating intermittent submersion of the tissue slice in the culture medium.

According to embodiments of the invention, the culture is in a tissue culture plate.

According to embodiments of the invention, the culture medium comprises DMEM/F12.

According to embodiments of the invention, the culturing is effected for at least 4 or 5 days.

According to embodiments of the invention, the slice is in direct contact with the tissue culture insert.

According to embodiments of the invention, the tissue culture insert is a titanium grid insert.

According to embodiments of the invention, the cancer cells exhibit at least one of:
(i) sensitivity to the MEK inhibitor and the SRC inhibitor in an EVOK model as monitored by cell killing;
(ii) sensitivity to the MEK inhibitor and the SRC inhibitor in an EVOK model based on the level of MEK phosphorylation (pMEK), wherein a level below a predetermined or control is indicative of an efficacious treatment; and/or
(iii) sensitivity to the SRC inhibitor in an EVOK model based on the level of ERK1/2 phosphorylation (pERK1/2), wherein a level below a predetermined or control is indicative of an efficacious treatment.

According to embodiments of the invention, the MEK inhibitor is selected from the group consisting of trametinib and selumetinib.

According to embodiments of the invention, the SRC inhibitor is selected from the group consisting of bosutinib, dasatinib, saracotinib, ponatinib, TPX-0022 and 1-NM-PP1.

According to embodiments of the invention, the method further comprises treatment with Folfox or Folfiri.

According to embodiments of the invention, the the CRC is selected from the group consisting of locally advanced, metastatic CRC and a recurrent disease.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

### BRIEF DESCRIPTION OF THE SEVERAL VIEWS OF THE DRAWING(S)

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:

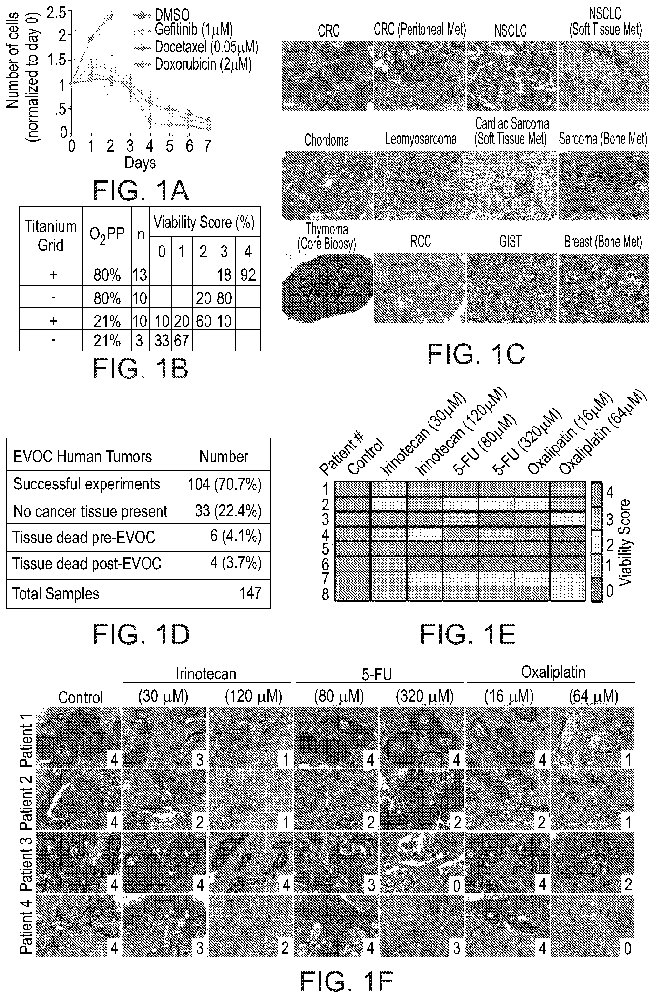

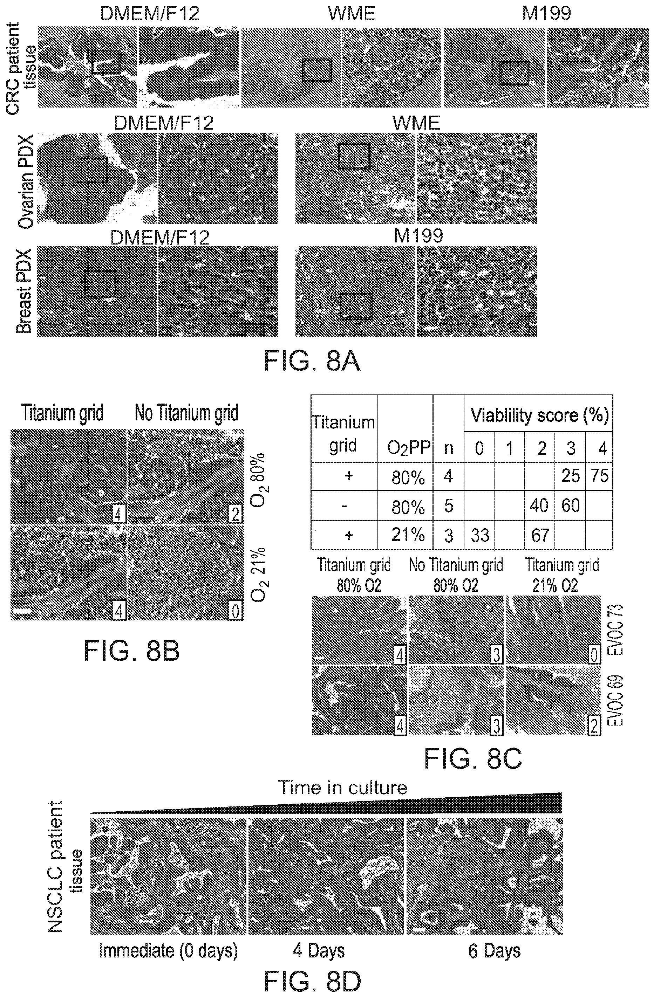

FIGs. 1A-F shows optimizing conditions for robust and versatile *ex vivo* organ culture. A, GFP-positive PC9 cells were treated with different drugs or with DMSO control, and counted daily by automated microscopy. Error bars represent standard deviation calculated from four experiments, each done in quadruplicates. B, Twenty-four experiments performed on a total of 2 PDX mouse models and 5 CRC human tissue samples, in which H&E staining of histological slices from the tumors were used to determine the viability of the cancer cells after five days in culture. Each experiment was performed in duplicates. A scoring system was used to categorize the percentage of viable cancer cells: 0 for less than 10% viable cancer cells, 1 for 10-30%, 2 for 31-60%, 3 for 61-90%, and 4 for more than 90%. Examples of histological slices can be seen in Figure 8C, Example of twelve human cancer tissues after 5 days of *ex vivo* culture. Scale bar 40µM. D, Table summarizing the different causes that led to EVOC culture failure in 43 of 147 human tumors tested. Successful experiments refer to a viability score of at least 3 when no drug was applied. Scores were determined by evaluating at least two slices from each tumor. E, Heatmap demonstrating the variability in the response of eight human CRC tumors to different drugs after five days of culture (of which the tissues were treated for four days). Control tissues were treated with DMSO. At least two slices from each tumor were evaluated for each tested condition. F, Representative histological sections stained with H&E for patients 1 to 4 in panel (E). Viability scores are depicted in the lower right corners. Scale bar 50µM. (GIST - gastrointestinal stromal tumor, Met - metastasis, NSCLC - non-small cell lung cancer, RCC - renal cell carcinoma, 5-FU - 5-Fluorouracil, DMSO - dimethyl sulfoxide).

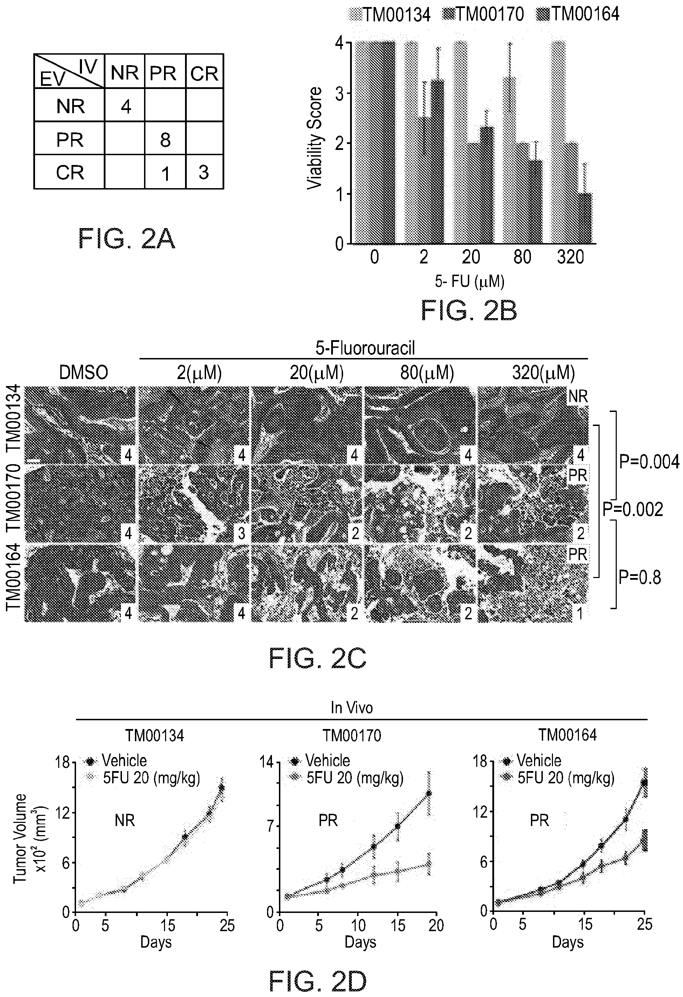

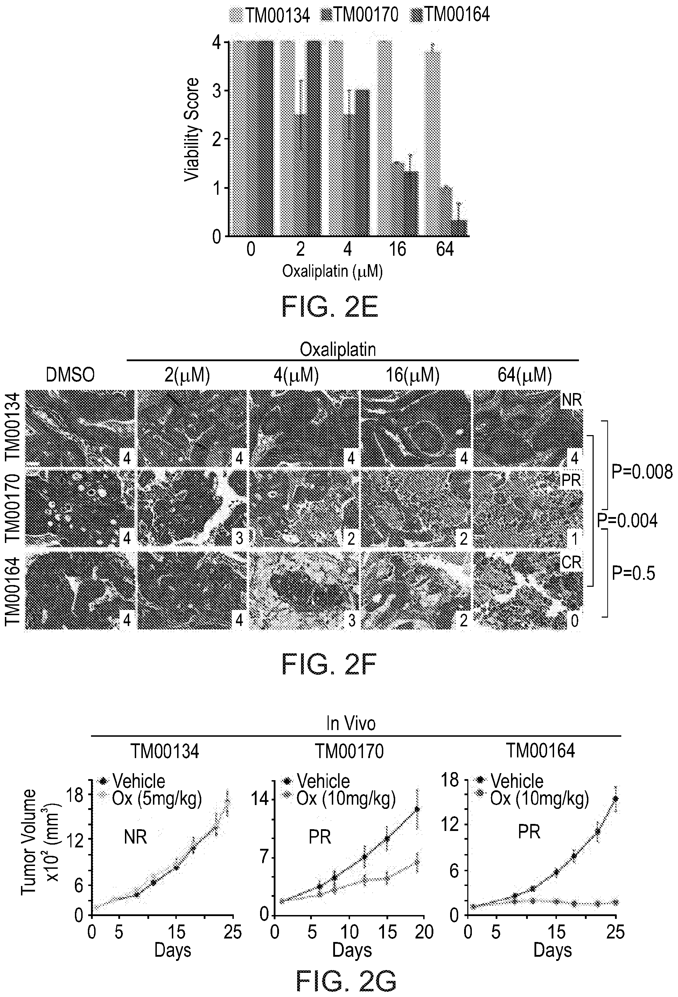

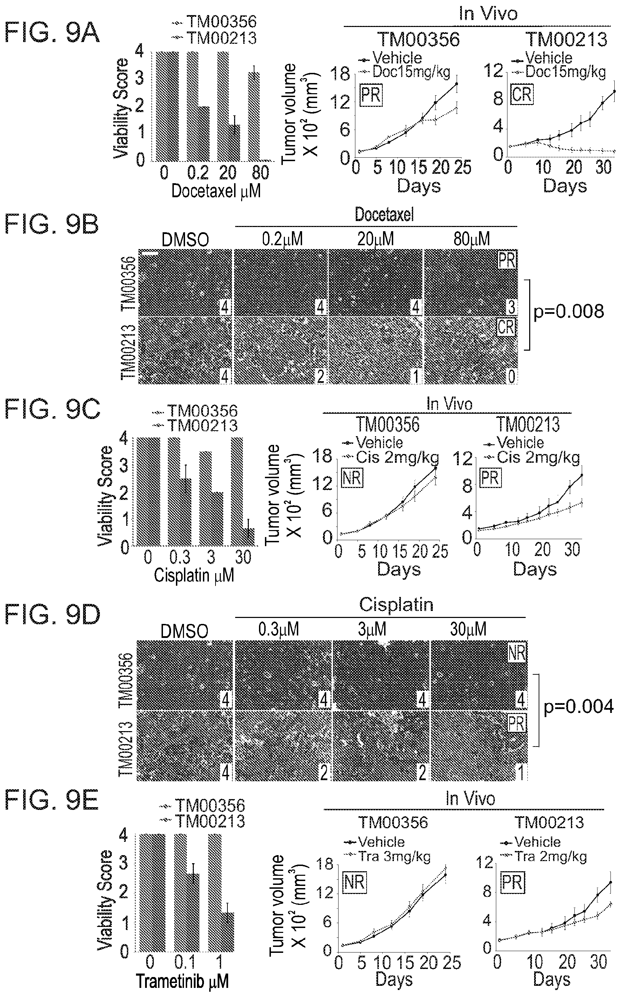

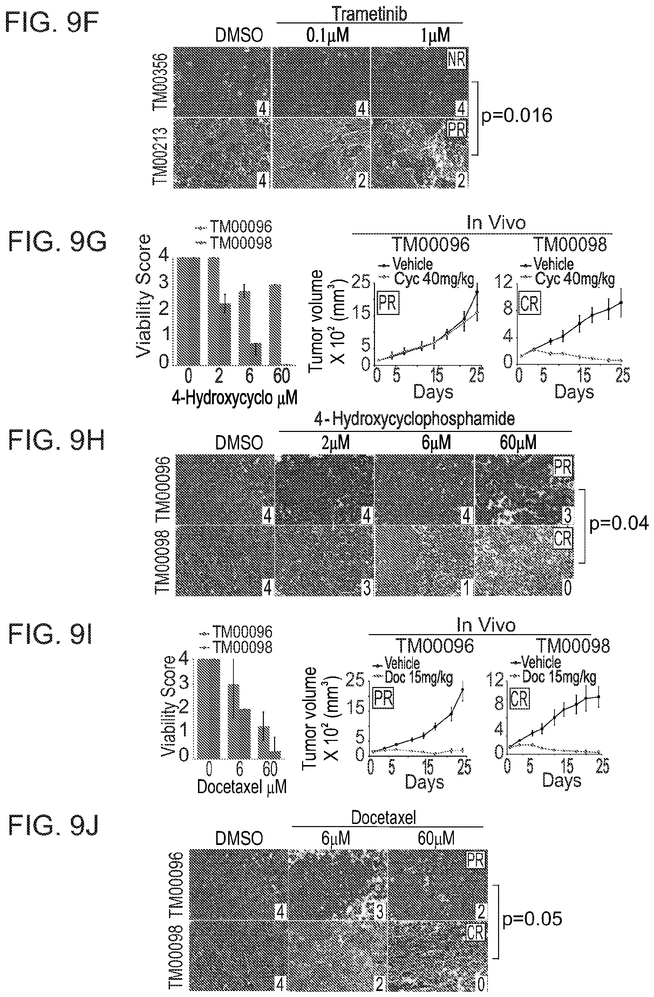

FIGs. 2A-G shows that EVOC can predict the response of PDX tumors to treatment. A, Table summarizing the *ex vivo* (EV) vs. *in vivo* (IV) response to drugs across 16 PDX models of colon, breast, and lung cancers. *Ex vivo* experiments were repeated at least twice, each evaluating at least two tumor slices and were scored as follows: NR - no response (viability score 4); PR - partial response (viability scores 1-3); CR - complete response (viability score 0). *In vivo* experiments were scored by the percent reduction in fold change of the tumor growth between the treated and untreated control mice groups as follows: NR - No response (<30% reduction); PR - partial response (30-100% reduction); CR - complete or near-complete response (>100% reduction). B,E, Bar plots demonstrating the viability scores of three CRC PDX tumors treated *ex vivo* for 4 days, with a range of concentrations of 5-FU or Oxaliplatin. Bar errors represent the standard deviation of two to three biological repeats, each evaluating at least two slices. C,F, Representative histological sections of tissues from (B) and (E) stained with H&E. Viability scores depicted in the lower right corners. p values were calculated using a two-sided rank test, comparing the viability scores of all drug concentrations tested across all biological repeats. D,G, Growth curves representing the *in vivo* response of tumors to 5-FU or oxaliplatin or vehicle control. Data were generated by the Jackson Laboratories. Error bars represent standard error of the mean. Scale bars 50µM. Figures 9A-J present data for the additional 10 experiments that are not presented in (B-G).

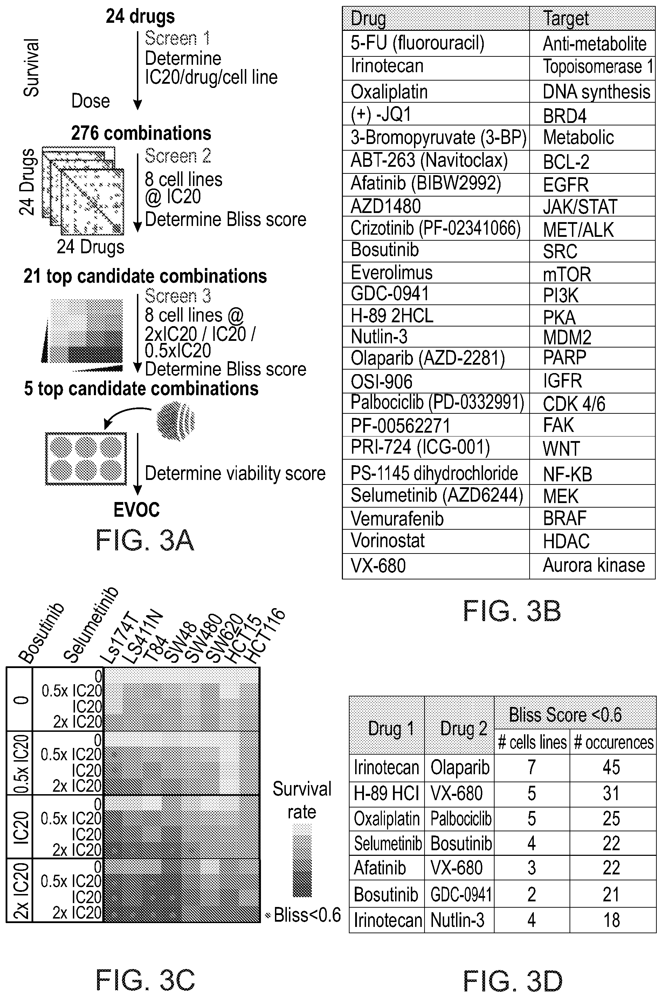

FIGs. 3A-D shows *In vitro* screens of drug combinations identify multiple synergistic pairs in CRC cell lines. A, Scheme depicting the three *in vitro* screens prioritizing synergistic drug pairs across human CRC cell lines. B, List of the 24 drugs tested and their primary targets. C, Heatmap depicting the survival rate after treatment with bosutinib and selumetinib in all eight CRC cell lines. Each drug was tested in three concentrations (0.5xIC20, IC20, and 2xIC20) resulting in nine combinations tested for each drug pair (screen 2 - see figure 1A). Dots indicate drug concentrations where synergy was observed (Bliss score<0.6). Results were averaged from four technical repeats. D, Table listing the seven top synergistic drug combinations based on screen 3. For each drug pair, the table shows the number of cell lines in which it was synergistic (Bliss score <0.6) in at least one of the nine tested combinations. The total number of occurrences in which the bliss score was < 0.6 out of the possible 72 occurrences (9 drug concentrations X 8 cell lines) is also depicted.

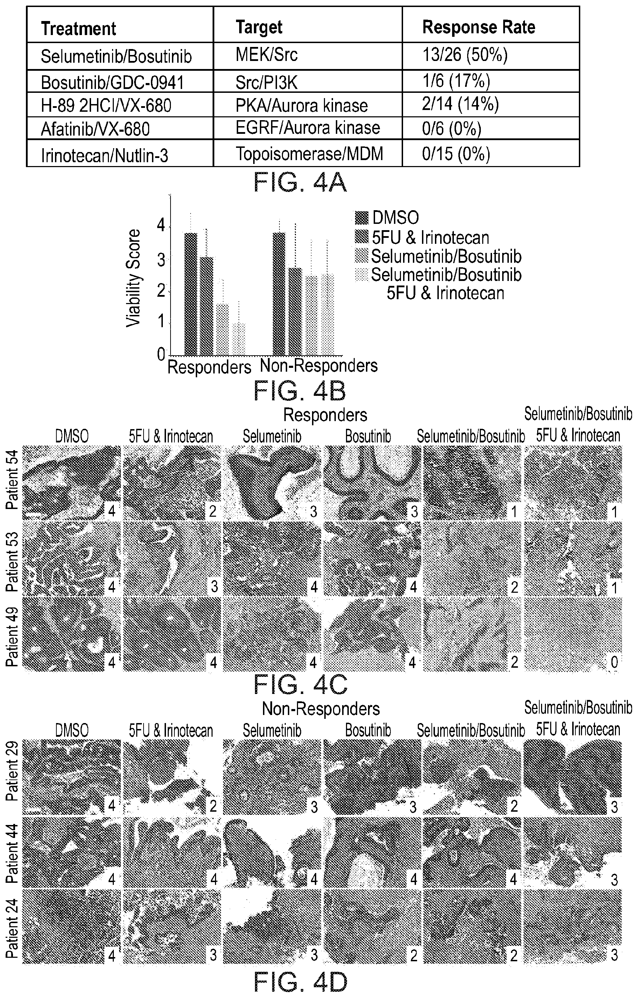

FIGs. 4A-D shows *Ex vivo* screening identifies the dual inhibition of MEK and Src as effective in a subset of human CRCs. A, Five drug combinations were tested *ex vivo* on human CRC tumors. Drugs (or DMSO control) were added 16-20 hours after the slicing of the tumors, and tissue slices were fixed after four complete days of treatment. In each condition, at least two tissue slices were evaluated. B, , Bar graph summarizing the viability of 26 human CRC tumors in response to ex vivo treatment with 5-FU and Irinotecan, selumetinib and bosutinib, or the combination of all four drugs. Responders refers to tumors that responded to the MEK/Src dual inhibition and non-responders to those that did not (raw data not shown). Error bars represent standard error. The p values were determined using a two-tailed Wilcoxon matched-pairs signed rank test. C,D, Representative histological sections stained with H&E of CRC tumors that responded (C) or did not respond (D) to MEK/Src dual inhibition. The effect of 5-FU and irinotecan with or without bosutinib and selumetinib is also shown. Scale bar 100µM. A scoring system was used to categorize the percentage of viable cancer cells: 0 for less than 10% viable cancer cells, 1 for 10-30%, 2 for 31-60%, 3 for 61-90%, and 4 for more than 90%. MEK, mitogen-activated protein kinase kinase; Src, proto-oncogene tyrosine-protein kinase Src; 5-FU, 5-Fluorouracil; EGFR, epidermal growth factor receptor; PKA, protein kinase A; MDM2, Mouse double minute 2 homolog; H-89 2HCl, H-89 dihydrochloride; PI3K, phosphoinositide 3-kinase; DMSO, dimethyl sulfoxide.

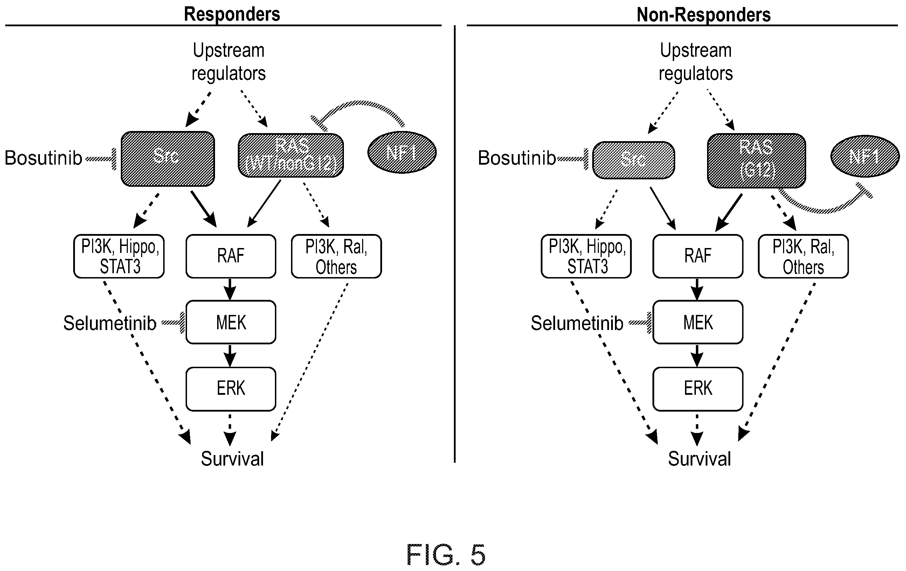

FIG. 5 shows a proposed model of the response to MEK/Src inhibition in CRC. Only a subset of CRC patients have high levels of pSrc in their tumor cells. In these patients, most of the flux in the MAPK pathway is driven by Src. Consequently, inhibition of Src has a greater effect on pERK in these patients than in non-responsive patients. Nevertheless, treatment with Srci alone is insufficient to kill the cancer cells, and the addition of MEK inhibitors to attenuate the low residual pERK resulting from non-Src-dependent upstream signaling is needed to kill the cancer cells. There is an anti-correlation between the presence of *KRAS* G12 mutations and response to MEK/Src inhibition. This may be the result of the unique ability of KRAS G12 mutations to inhibit the RAS family negative regulator NF1, resulting in high Ras-dependent signaling. Strong negative feedback signaling that results from the high KRAS G12 signaling may drive low Src activity and lack of response to MEK/Src inhibition in these tumors. By contrast, in tumors with wild-type KRAS or non-*KRAS* G12 mutations, NF1 may control the activity of ras family proteins, allowing Src activation and the consequent sensitivity to MEK/Src inhibition. NF1 - Neurofibromin 1, MEK - mitogen-activated protein kinase kinase, ERK - extracellular signal-regulated kinase, PI3K - phosphoinositide 3-kinase.

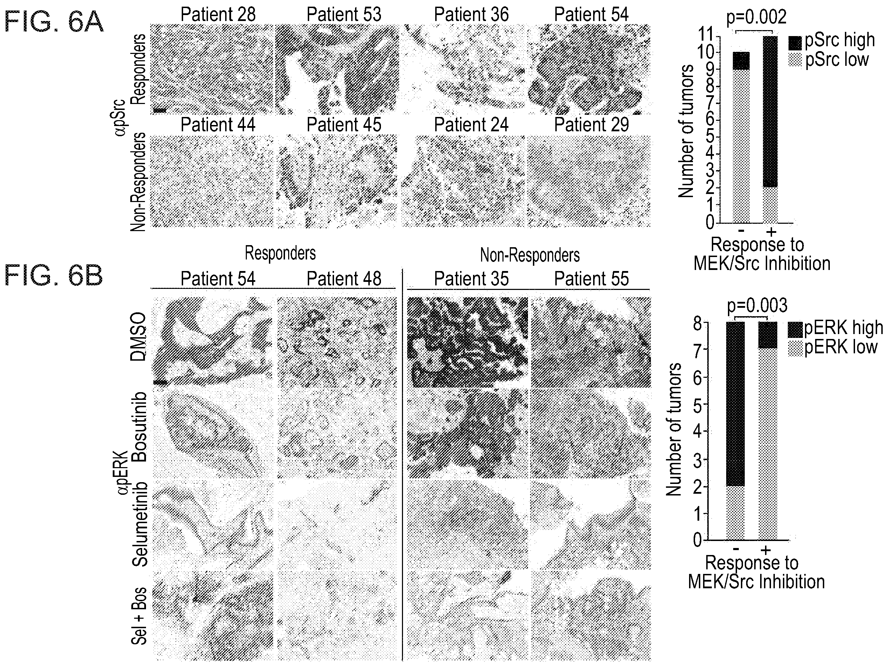

FIGs. 6A-B shows that high pSrc and inhibition of pERK by the Srci bosutinib are both predictive biomarkers of response to MEK/Src inhibition in CRC. A, Immunohistochemistry staining with anti-pSRC (Y416) antibody of CRC tumors from both responders and non-responders to MEK/Src inhibition. Bar plot on the right summarizes results of 21 tumors for which tissue was available for staining. P value was determined by Fisher's exact test (two-tailed). Scale bar 40µM. B, Immunohistochemistry staining with anti-pERK1/2 (T202/Y204) antibody of CRC tumors treated *ex vivo* as labeled. Bar plot on the right summarizes results of 16 tumors from both responders and non-responders for MEK/Src inhibition for which tissue was available for staining. P value was determined by Mann Whitney test (two-tailed). Scale bar 100uM. The intensity of pSrc and pERK in the samples was scored from 0-3 by a pathologist blinded to treatment results. Scores of 0 or 1 were designated as low pSrc and pERK whereas scores 2 or 3 were designated as high pSrc and pERK. Score of 0 - no staining; score of 1 - weak and focal cytoplasmic staining; score of 2 - weak and diffuse or strong and focal cytoplasmic staining; score of 3 - strong and diffuse cytoplasmic staining.

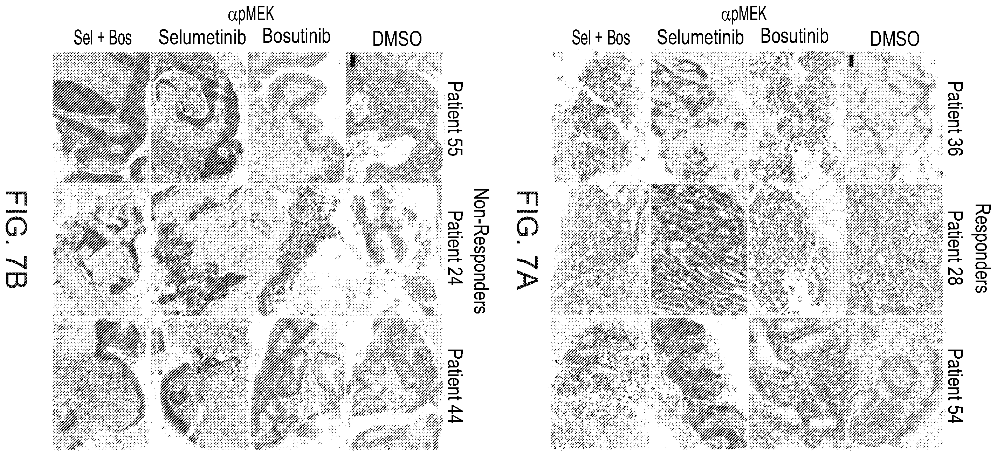

FIGs. 7A-B shows that feedback phosphorylation of MEK as a result of MEK inhibition is abrogated by the addition of Srci in CRC tumors that are sensitive to MEK/Src inhibition. A,B, Immunohistochemistry staining with anti-pMEK1/2 (S221) antibody of CRC tumors treated *ex vivo* as labeled. Three tumors that respond to MEK/Src inhibition (A) and three that do not respond (B) were selected for staining. Scale bar 40µM.
FIGs. 8A-D shows optimization of culture conditions for EVOC. a, An example of H&E staining of colorectal cancer from a patient (top row), ovarian cancer tissue (middle row) and breast cancer tissue (bottom row) from PDX mouse models cultured in different media for five days. Boxes mark the regions that were selected for magnification on the right. Representative pictures from two experiments are presented. Scale bar 50µM in low magnifications and 20µM in high magnifications. b, Examples of H&E staining of histological slices of ovarian PDX tumors used to determine the viability of cancer cells after five days in culture under the different conditions as labeled. A scoring system was used to categorize the percentage of viable cancer cells: 0 for less than 10% viable cancer cells, 1 for 10-30%, 2 for 31-60%, 3 for 61-90%, and 4 for more than 90%. c, Upper panel summarizes experiments performed using 5 human CRC tissues, in which H&E staining was used to determine the effect of using a grid or high oxygen partial pressure on the viability of cancer cells after five days in culture. The bottom panel shows representative histological slices from two of the tumors in the upper panel. Each experiment was performed using at least two tissue slices for each condition. Scale bar 40µM. d, H&E staining of a human non-small cell lung cancer (NSCLC) tumor that was sliced and immediately fixed or cultured on a titanium grid in 80% O₂PP for 4 or 6 days. Scale bar 50µM. O₂PP, oxygen partial pressure; DMSO, dimethyl sulfoxide.
FIGs. 9A-J show that EVOC can predict the response of PDX tumors to treatment. a,c,e, left panels, Bar plots demonstrating the viability scores of two NSCLC PDX tumors treated *ex vivo* for 4 days with a range of concentrations of docetaxel, cisplatin or trametinib. Bar errors represent the standard deviation of two to three experiments, each condition within an experiment was evaluated in at least two slices of tissue. Right panel, growth curves representing the *in vivo* response of tumors to docetaxel, cisplatin or trametinib, or vehicle control. *In vivo* data was generated by the Jackson Laboratories. Error bars represent standard error of the mean. b,d,f, Representative histological sections of tissues from (a), (c) and (e) stained with H&E. g,i, Left panel, bar plots demonstrating the viability scores of two breast cancer PDX tumors treated *ex vivo* for 4 days with a range of concentrations of 4-hydroxycyclophosphamide (active metabolite of the pro-drug cyclophosphamide) and docetaxel. Error bars represent the standard deviation of two to three experiments, each evaluating at least two slices of tissue for each condition. g,i, Right panel, growth curves representing the *in vivo* response of tumors to cyclophosphamide, docetaxel, or vehicle control. *In vivo* data was generated by the Jackson Laboratories. Error bars represent standard error of the mean. h,j, Representative histological sections of tissues from (g) and (i) stained with H&E. The p values were calculated using a one-sided rank test, comparing the viability scores of all drug concentrations tested across all experiments. Viability scores were generated as detailed in Figures 1A-F: NR - no response (viability score 4). PR - partial response (viability scores 1-3). CR - complete response (viability score 0). In vivo experiments were scored by the percent reduction in fold change of the tumor growth between the treated and untreated control mice groups as follows: NR - No response (<30% reduction); PR - partial response (30-100% reduction); CR - complete or near-complete response (>100% reduction). Scale bars 100µM. Doc, docetaxel; Cis, cisplatin; Tra, trametinib; Cyc, cyclophosphamide, DMSO, dimethyl sulfoxide.

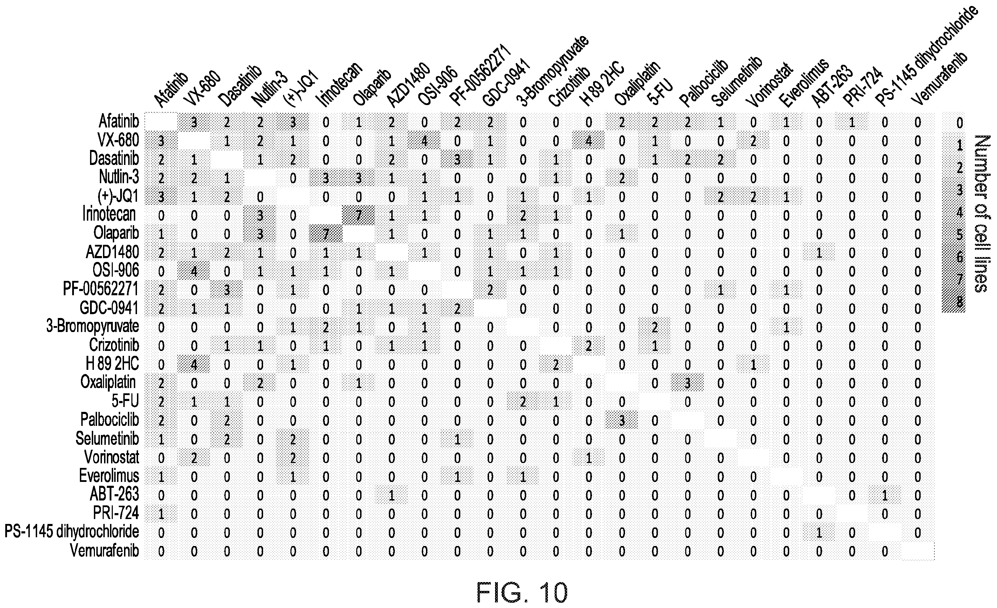

FIG. 10 shows *in vitro* combinations for drug screen. Two hundred and seventy six drug combinations were tested on eight colorectal cancer cell lines (Figure 3A - screen 2). Numbers represent the number of cell lines for which the combination of drugs was synergistic as determined by a Bliss score < 0.6.

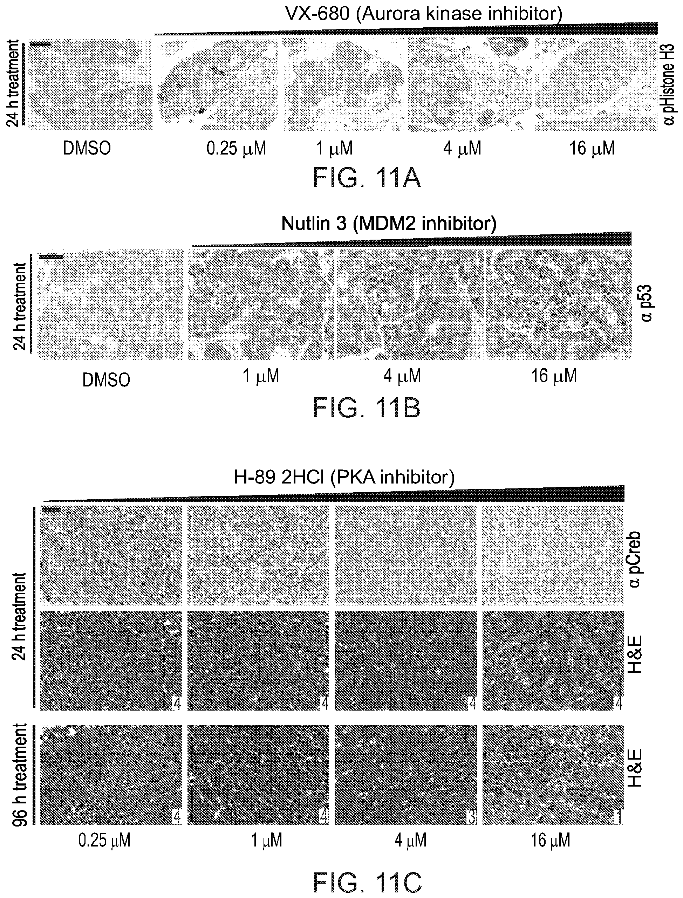

FIGs. 11A-C show examples of calibrating drug doses for *ex vivo* organ culture using colorectal cancer PDX tumors. a,b,c. Representative IHC and H&E staining of tumors from the CRC PDX model TM00170 (a,b) or TM00164 (c) cultured *ex vivo* with increasing concentrations of different drugs. Tissues were fixed after 24-96 hours of treatment, as indicated in the figure. Viability scores are depicted in the right lower corners of the H&E stained slides. Scale bars 50µM (a,b) 40µM (c). At least two tissue slices were evaluated for each condition. PKA, protein kinase A; MDM2, Mouse double minute 2 homolog; H-89 2HCl, H-89 dihydrochloride; h, hour; DMSO, dimethyl sulfoxide.

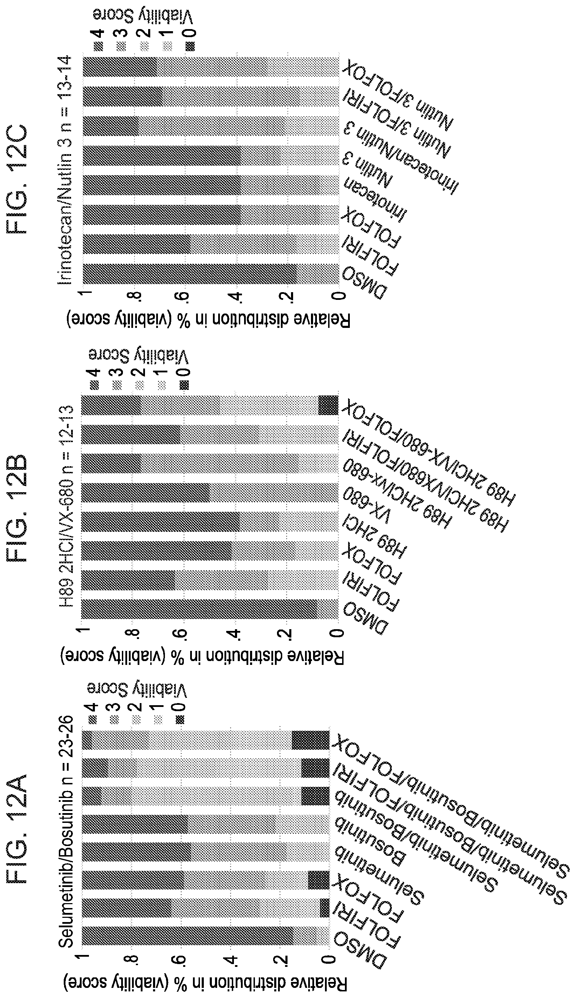

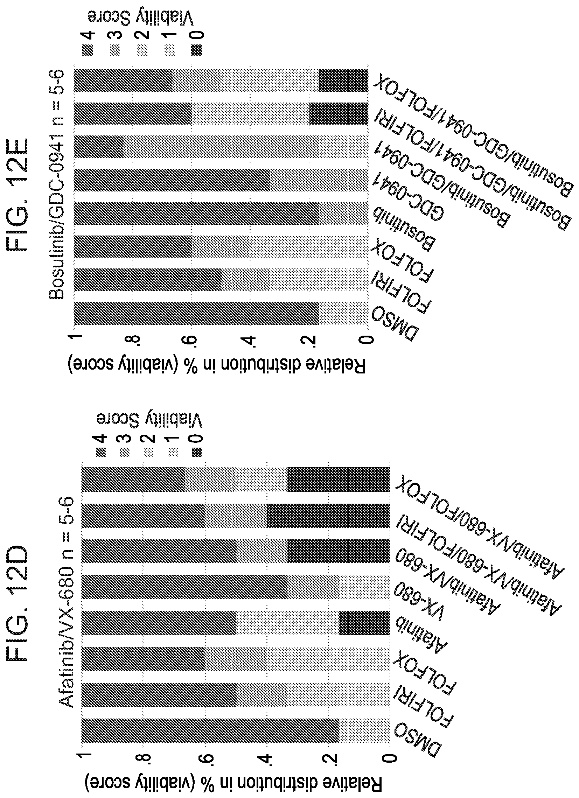

FIGs. 12A-E show the response of CRC human tumors to five combinatory treatments. All tumors were treated ex vivo for 4 days in duplicate with either DMSO control, or drugs as indicated. Viability of the tissue slices as reflected in H&E staining was scored from 0 to 4, by a pathologist blinded to the treatments: 0 for less than 10% viable cancer cells, 1 for 10-30%, 2 for 31-60%, 3 for 61-90%, and 4 for more than 90%. FOLFOX, 5-fluorouracil/oxaliplatin; FOLFIRI, 5-fluorouracil/irinotecan; DMSO, dimethyl sulfoxide.

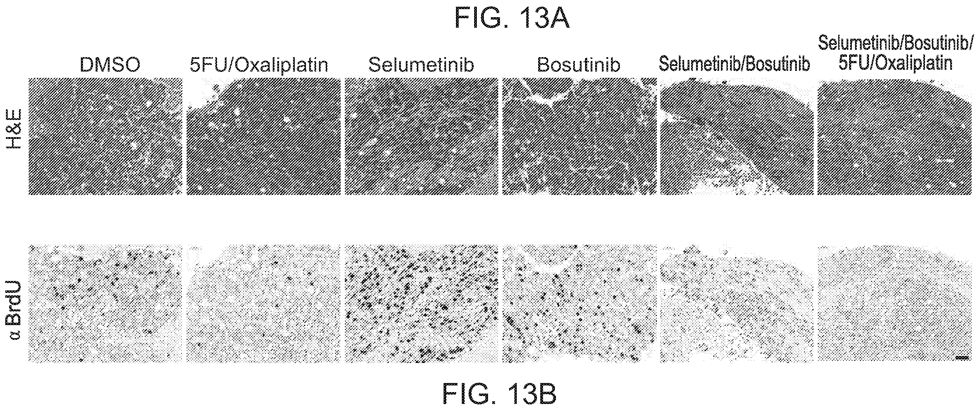

FIGs. 13A-B show the effect of MEK/Src inhibition on the proliferation of cancer cells. Example of CRC tumor from patient 28 - a responder to dual inhibition of MEK and Src. Drugs (or DMSO control) were added 16-20 hours after the slicing of the tumor, and tissue slices were fixed after four complete days of treatment. Bromodeoxyuridine (BrdU) was added to all slices 18h hours before the end of the experiment. In each condition, at least two tissue slices were evaluated. a, Representative histological sections stained with H&E. b, Representative histological slices from the same EVOC slices in (a), stained for anti-BrdU. Note that proliferation is evident as measured by the incorporation of BrdU when the tissue is treated with either selumetinib or bosutinib, but is markedly reduced when treated with both drugs. Scale bar 50µM.

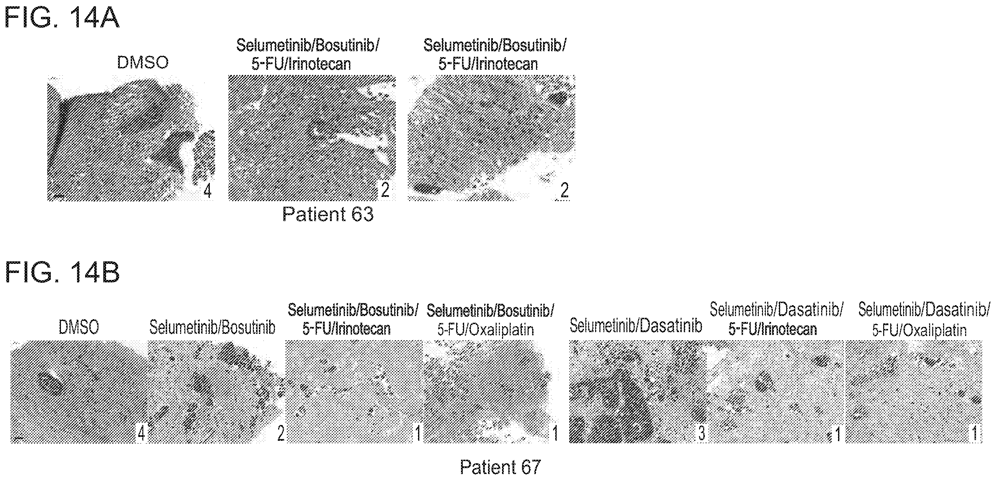

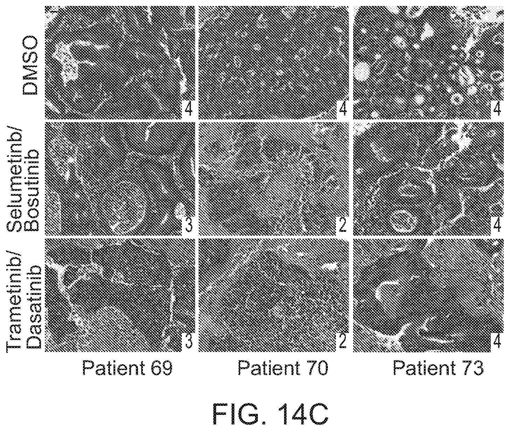

FIGs. 14A-C show that thee Src inhibitors dasatinib and bosutinib and the MEK inhibitors trametinib and selumetinib show similar response when tested ex vivo on human colorectal tumors. Drugs (or DMSO control) were added 16-20 hours after the slicing of the tumor, and tissue slices were fixed after four complete days of treatment. In each condition, at least two tissue slices were evaluated a,b. The effect of dasatinib combined with selumetinib is compared to the effect of bosutinib combined with selumetinib c, The effect of trametinib combined with dasatinib is compared to the combination of selumetinib and bosutinib. Viability scores are depicted in the right lower corners. Scale bar 20µM. 5-FU, 5-fluorouracil; DMSO, dimethyl sulfoxide.

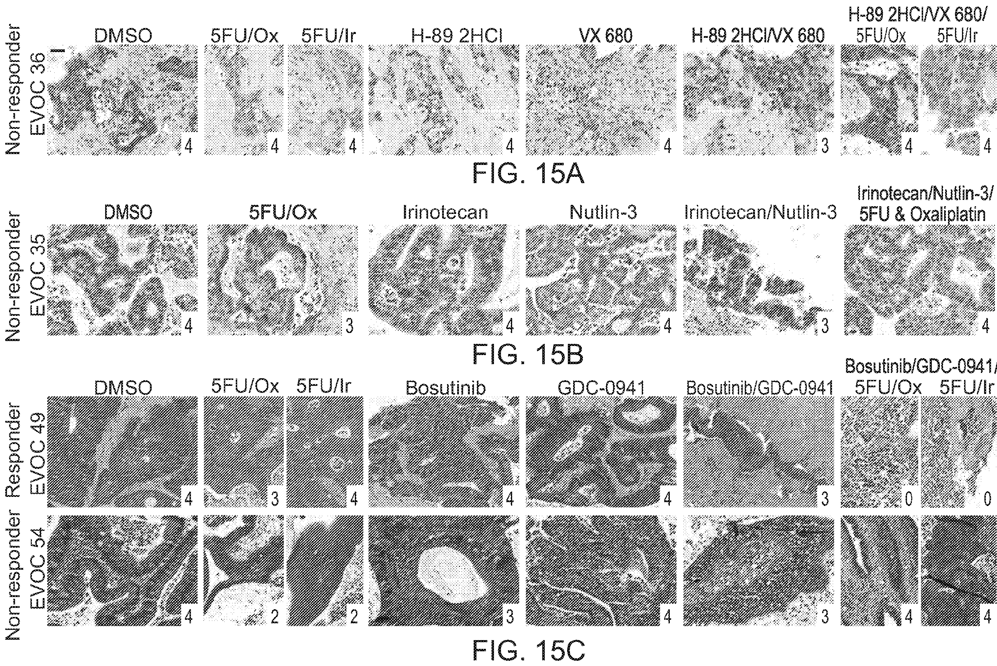

FIGs. 15A-C show additional examples of the response of human colorectal (CRC) tumors to several drug combinations tested in EVOC. a,b,c. Drugs (or DMSO control) were added 16-20 hours after the slicing of the tumor, and tissue slices were fixed after four complete days of treatment. In each condition, at least two tissue slices were evaluated. Viability scores are depicted in the right lower corners. Scale bar 40µM. 5-FU, 5-fluorouracil; Ox, oxaliplatin; Ir, irinotecan; H-89 2HCl, H-89 dihydrochloride; DMSO, dimethyl sulfoxide.

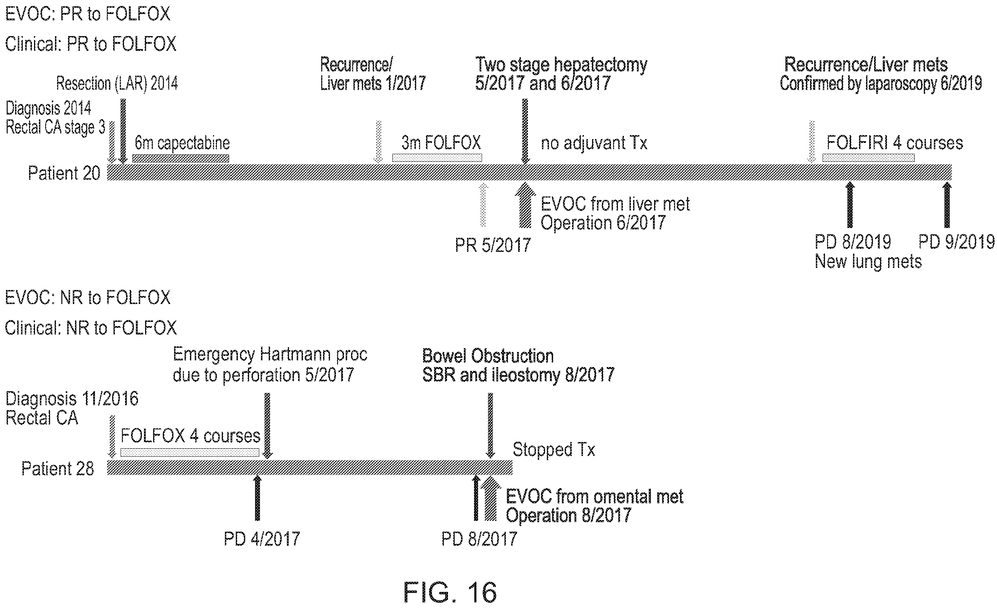

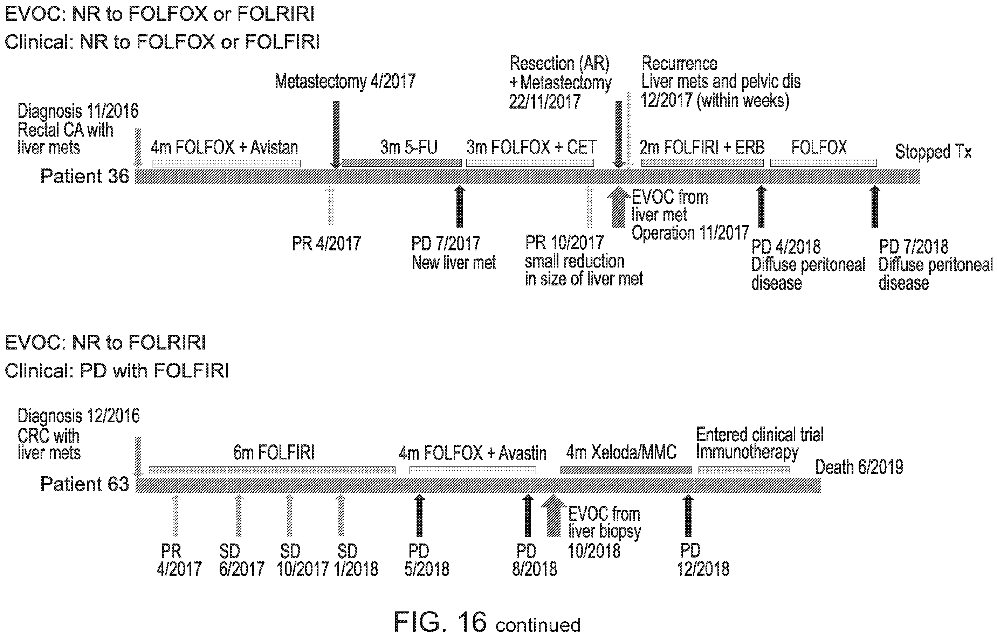

FIG. 16 shows *ex vivo* response to chemotherapy correlates with the clinical course in selected patients. From the 28 patients that entered the study to test the response of their CRC tissue to chemotherapy and targeted dual therapy, four patients were available for evaluation and comparison to ex vivo data. Clinical evaluation of disease state was determined by PET/CT and interpreted by the treating physician independently and without knowledge of the results of the ex vivo study. FOLFOX, 5-fluorouracil and oxaliplatin; FOLFIRI, 5-fluorouracil and irinotecan; MMC, mitomycin C; LAR, low anterior resection; mets, metastasis; AR, anterior resection; CA, carcinoma; CRC, colorectal carcinoma; CET, cetuximab; ERB, erlotinib; Tx, treatment; PR, partial response (green arrows); SD, stable disease (grey arrows); PD, progressive disease (black arrows). Light blue arrows indicate time of diagnosis. Royal blue arrows indicate operative intervention. Yellow arrows indicate recurrence of disease. Red arrows indicate when tissue was obtained for ex vivo organ culture.

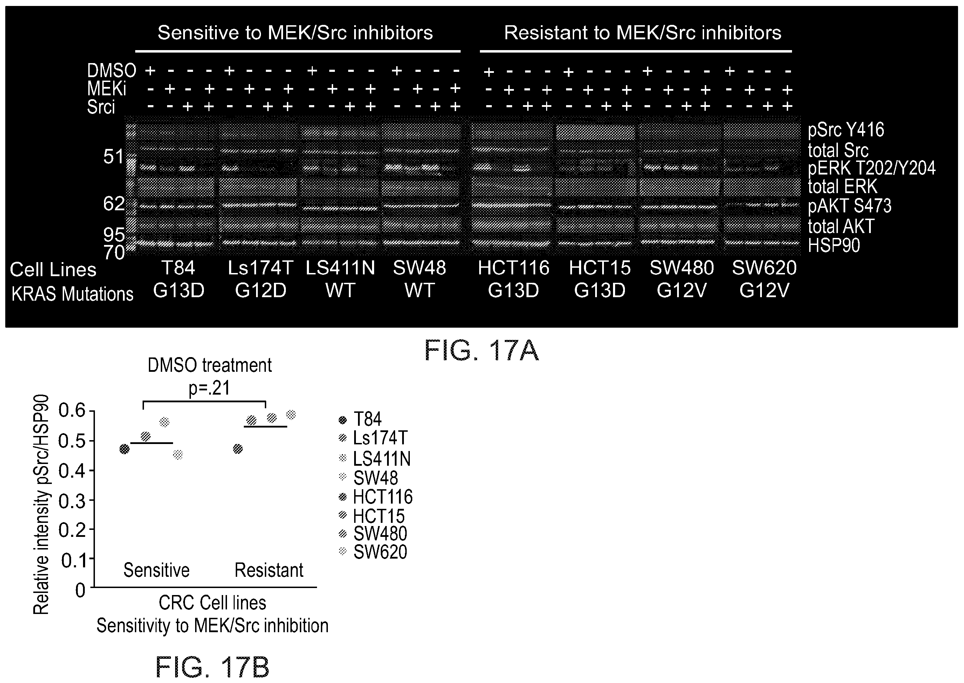

FIGs. 17A-B. Sensitivity to MEK and Src inhibition does not correlate with phosphorylation of Src in CRC cell lines. The eight CRC cell lines used in the *in vitro* screens (see Figures 3A-D) were seeded in 6 cm plates and treated as labeled for 24 hours and then collected for western blot evaluation. To determine the best drug dose for each cell line we utilized the extensive data from the in vitro screens: The IC20 was determined in the in vitro screen #1 and the most synergistic dose of the combined MEK/Src inhibition (selumetinib/bosutinib) (either 0.5xIC20, IC20 or 2XIC20) was determined in the in vitro screen #3. We used the most effective synergistic dose for each cell line. A, Western blot analysis of eight CRC cell lines as labeled. B, Phosphorylated Src signal intensity determined as a ratio of HSP90 (heat shock protein 90) signal intensity used as loading control. Lines represent average intensity for each group and p value = 0.21 as determined by two-tailed student t-test. The experiment was performed in 8 different cell lines (n=8 cell lines; n=1 biological repeat). MEK, mitogen-activated protein kinase kinase; ERK, extracellular signal-regulated kinase; Src, proto-oncogene tyrosine-protein kinase Src.

### DESCRIPTION OF SPECIFIC EMBODIMENTS OF THE INVENTION

The present invention, relates to colorectal cancer treatment.

Before explaining at least one embodiment of the invention in detail, it is to be understood that the invention is not necessarily limited in its application to the details set forth in the following description or exemplified by the Examples. The invention is capable of other embodiments or of being practiced or carried out in various ways.

The translation of pre-clinical studies to effective treatment protocols as well as the individual response of cancer patients to therapy is generally unpredictable. This may be due to the complex genetic makeup of tumor cells and their complex tumor microenvironment. To find novel clinically relevant drug combinations for colorectal cancer (CRC) the present inventors prioritized the top five synergistic combinations from a large *in vitro* screen for *ex vivo* testing on 29 freshly resected human CRC tumors. It was found that only one of these combinations, the combination of MEK and Src inhibition, was effective when tested *ex vivo.* Pre-treatment phosphorylated Src (pSrc) was identified as a predictive biomarker for MEK/Src inhibition but only in the absence of *KRAS*-G12 mutations. Other KRAS mutations did not impede the MEK/Sec sensitivity predicted by high pSrc. Overall, it is demonstrated the potential of MEK/Src inhibition as an effective drug combination in a pre-defined subset of CRC patients.

Thus, there is dislcosed a method of treating colorectal cancer (CRC), the method comprising administering to a subject in need thereof a therapeutically effective amount of a MEK inhibitor and a SRC inhibitor, wherein cancer cells of the subject express an amount of SRC p419 above a predetermined level and do not express a KRAS G12 mutation, thereby treating the CRC.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

As used herein, the term "treating" includes abrogating, substantially inhibiting, slowing or reversing the progression of a condition, substantially ameliorating clinical or aesthetical symptoms of a condition or substantially preventing the appearance of clinical or aesthetical symptoms of a condition.

The subject may be a healthy animal or human subject undergoing a routine well-being check-up. Alternatively, the subject may be at risk of having cancer (e.g., a genetically predisposed subject, a subject with medical and/or family history of cancer, a subject who has been exposed to carcinogens, occupational hazard, environmental hazard] and/or a subject who exhibits suspicious clinical signs of a malignant disease [e.g., blood in the stool or melena, unexplained pain, sweating, unexplained fever, unexplained loss of weight up to anorexia, changes in bowel habits (constipation and/or diarrhea), tenesmus (sense of incomplete defecation, for rectal cancer specifically), anemia and/or general weakness).

The term "colorectal cancer" refers a medical condition characterized by cancer of cells of the intestinal tract below the small intestine (i.e., the large intestine (colon), including the cecum, ascending colon, transverse colon, descending colon, sigmoid colon, and rectum.

The term "SRC Proto-Oncogene, Non-Receptor Tyrosine Kinase" abbreviated to SRC (also known as pp60src) (EC 2.7.10; SwissProt P12931) is a non-receptor tyrosine kinase involved in signal transduction in many biological systems and implicated in the development of human tumors. It is a member of the Src family of kinases.

As used herein "SRC p419" refers to phosphorylation of SRC (pSRC) at position 419, typically determined by immunofluorescent staining. It will be appreciated that antibodies that bind alternatively or additionally p419 on SRC can be used too.

In an embodiment, the pSrc staining can be scored according to the following scoring system (e.g., using the conditions described in the Examples section):
0: no staining
1: weak and focal cytoplasmic staining
2: weak and diffuse or strong and focal cytoplasmic staining
3: strong and diffuse cytoplasmic staining

Above a predetermined threshold means 2 or 3.

The term "KRAS" refers to nucleic acid sequences encoding a KRas protein, peptide, or polypeptide (e.g., KRAS transcripts, such as the sequences of KRAS Genbank Accession Nos. NM_033360.2 and NM_004985.3). KRAS, a member of the RAS family, is a key regulator of signaling pathways responsible for cell proliferation, differentiation, and survival. The term KRAS G12 mutation refers to a point mutation at glycine 12 of KRAS gene. Examples of such mutations include G12V, G12C, G12D, G12R and G12S. Other mutations are disclosed in Serebriiskii IG et al., Nat Commun. 2019 Aug 19;10(1):3722. doi: 10.1038/s41467-019-11530-0. PMID: 31427573; PMCID: PMC6700103, the contents of which are incorporated herein by reference.

The term "MEK," as used herein, means the family of mitogen/extracellular signal-regulated kinases (MEKs), and are kinase enzymes which phosphorylate mitogen-activated protein kinase (MAPK). MEK is also known as MAP2K and MAPKK. Isoforms of MEK include, but are not limited to, MEK1 and MEK2. As used herein, the term "MEK1" means mitogen/extracellular signal-regulated kinase-1 having the UniProt identifier MP2K1_HUMAN and encoded by the MAP2K1 gene. As used herein, the term "MEK2" means mitogen/extracellular signal-regulated kinase-2 having the UniProt identifier MP2K2_HUMAN and encoded by the MAP2K2 gene. Unless otherwise indicated, use of the term "MEK" may refer to MEK1, MEK2, or a combination thereof.

As used herein "MEK inhibitor" refers to an agent (e.g., nucleic acid, protein or small molecule) that inhibits e.g., catalytically (or by way of expression) the mitogen-activated protein kinase kinase enzymes MEK1 and/or MEK2. A MEK inhibitor prevents activation of ERK1/2 (p44/p42 MAPK) by MEK1/2.

Examples include, but are not limited to:
Binimetinib, Cobimetinib or XL518, Selumetinib, Trametinib (GSK1120212), PD-325901, CI-1040, PD035901, TAK-733.

According to a specific embodiment, the MEK inhibitor is Selumetinib or Trametinib.

As used herein "SRC inhibitor" refers to a substance (e.g., nucleic acid, protein or small molecule) that inhibits e.g., catalytically (or by way of expression) the tyrosine phosphorylation activity of SRC. Examples include, but are not limited to, Dasatinib and Bosutinib, Saracotinib, Ponatinib, TPX-0022, 1-NM-PP1.

In order to determine whether cancer cells express SRC p419 above a predetermined level and do not express a KRAS G12 mutation, cancer cells or tissue may be obtained from the subject by biopsy, laparoscopy, endoscopy or as xenograft or any combinations thereof.

Methods of detecting sequence alteration on the RNA level are known in the art and include for example Northern blot analysis and RT-PCR.

Methods of detecting sequence alteration at the protein level are known in the art and include for example Western Blot analysis, FACS analysis, immunohistochemical analysis. Antibodies may be used which bind specifically to SRC p419 and the G12 mutation of KRAS.

To determine sequence alterations in the KRAS gene, a DNA sample is obtained from the cancer cells.

The sequence alteration of some embodiments of the invention can be identified using a variety of methods. One option is to determine the entire gene sequence of a PCR reaction product. Alternatively, a given segment of nucleic acid may be characterized on several other levels. At the lowest resolution, the size of the molecule can be determined by electrophoresis by comparison to a known standard run on the same gel. A more detailed picture of the molecule may be achieved by cleavage with combinations of restriction enzymes prior to electrophoresis, to allow construction of an ordered map. The presence of specific sequences within the fragment can be detected by hybridization of a labeled probe, or the precise nucleotide sequence can be determined by partial chemical degradation or by primer extension in the presence of chain-terminating nucleotide analogs. Exemplary methods for determining the sequence of KRAS include mismatch detection assays based on polymerases and ligases, ligase/polymerase-mediated genetic Bit analysis, hybridization assay methods, Single-Strand Conformation Polymorphism (SSCP), Dideoxy fingerprinting (ddF), Pyrosequencing analysis and Acycloprime analysis (Perkin Elmer, Boston, Massachusetts, USA).

As used herein "a predetermined level" refers to a threshold which is typically determined with respect to a control obtained from non-responsive colorectal tumors to the treatment with MEK inhibitor and SRC inhibitor, in a statistically significant manner.

As used herein "above a predetermined level" refers to at least 10 %, 20 %, 30 %, 40 %. 50 %, 60 %, 70 %, 80 %, 90 % or more 1.5 fold, 2, fold, 5 fold 10 fold or more.

As used herein "below a predetermined level" refers to at least 10 %, 20 %, 30 %, 40 %. 50 %, 60 %, 70 %, 80 %, 90 % or more 1.5 fold, 2, fold, 5 fold 10 fold or more.

The MEK inhibitors and the SRC inhibitors of embodiments of the invention can be administered to an organism per se, or in a pharmaceutical composition where it is mixed with suitable carriers or excipients.

As used herein a "pharmaceutical composition" refers to a preparation of one or more of the active ingredients described herein with other chemical components such as physiologically suitable carriers and excipients. The purpose of a pharmaceutical composition is to facilitate administration of a compound to an organism.

Herein the term "active ingredient" refers to the agent that inhibits the synthesis and/or activity of cortisol which is accountable for the biological effect.

Hereinafter, the phrases "physiologically acceptable carrier" and "pharmaceutically acceptable carrier" which may be interchangeably used refer to a carrier or a diluent that does not cause significant irritation to an organism and does not abrogate the biological activity and properties of the administered compound. An adjuvant is included under these phrases.

Herein the term "excipient" refers to an inert substance added to a pharmaceutical composition to further facilitate administration of an active ingredient. Examples, without limitation, of excipients include calcium carbonate, calcium phosphate, various sugars and types of starch, cellulose derivatives, gelatin, vegetable oils and polyethylene glycols.

Techniques for formulation and administration of drugs may be found in "Remington's Pharmaceutical Sciences," Mack Publishing Co., Easton, PA, latest edition, which is incorporated herein by reference.

Suitable routes of administration may, for example, include oral, rectal, trans-mucosal, especially trans-nasal, intestinal or parenteral delivery, including intramuscular, subcutaneous and intramedullary injections as well as intrathecal, direct intraventricular, intracardiac, e.g., into the right or left ventricular cavity, into the common coronary artery, intravenous, intraperitoneal, intranasal, or intraocular injections.

Conventional approaches for drug delivery to the central nervous system (CNS) include: neurosurgical strategies (e.g., intracerebral injection or intracerebroventricular infusion); molecular manipulation of the agent (e.g., production of a chimeric fusion protein that comprises a transport peptide that has an affinity for an endothelial cell surface molecule in combination with an agent that is itself incapable of crossing the BBB) in an attempt to exploit one of the endogenous transport pathways of the BBB; pharmacological strategies designed to increase the lipid solubility of an agent (e.g., conjugation of water-soluble agents to lipid or cholesterol carriers); and the transitory disruption of the integrity of the BBB by hyperosmotic disruption (resulting from the infusion of a mannitol solution into the carotid artery or the use of a biologically active agent such as an angiotensin peptide). However, each of these strategies has limitations, such as the inherent risks associated with an invasive surgical procedure, a size limitation imposed by a limitation inherent in the endogenous transport systems, potentially undesirable biological side effects associated with the systemic administration of a chimeric molecule comprised of a carrier motif that could be active outside of the CNS, and the possible risk of brain damage within regions of the brain where the BBB is disrupted, which renders it a suboptimal delivery method.

Alternately, one may administer the pharmaceutical composition in a local rather than systemic manner, for example, via injection of the pharmaceutical composition directly into a tissue region of a patient.

The term "tissue" refers to part of an organism consisting of cells designed to perform a function or functions. Examples include, but are not limited to, brain tissue, retina, skin tissue, hepatic tissue, pancreatic tissue, bone, cartilage, connective tissue, blood tissue, muscle tissue, cardiac tissue brain tissue, vascular tissue, renal tissue, pulmonary tissue, gonadal tissue, hematopoietic tissue.

Pharmaceutical compositions of some embodiments of the invention may be manufactured by processes well known in the art, e.g., by means of conventional mixing, dissolving, granulating, dragee-making, levigating, emulsifying, encapsulating, entrapping or lyophilizing processes.

Pharmaceutical compositions for use in accordance with some embodiments of the invention thus may be formulated in conventional manner using one or more physiologically acceptable carriers comprising excipients and auxiliaries, which facilitate processing of the active ingredients into preparations which, can be used pharmaceutically. Proper formulation is dependent upon the route of administration chosen.

For injection, the active ingredients of the pharmaceutical composition may be formulated in aqueous solutions, preferably in physiologically compatible buffers such as Hank's solution, Ringer's solution, or physiological salt buffer. For transmucosal administration, penetrants appropriate to the barrier to be permeated are used in the formulation. Such penetrants are generally known in the art.

For oral administration, the pharmaceutical composition can be formulated readily by combining the active compounds with pharmaceutically acceptable carriers well known in the art. Such carriers enable the pharmaceutical composition to be formulated as tablets, pills, dragees, capsules, liquids, gels, syrups, slurries, suspensions, and the like, for oral ingestion by a patient. Pharmacological preparations for oral use can be made using a solid excipient, optionally grinding the resulting mixture, and processing the mixture of granules, after adding suitable auxiliaries if desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethyl-cellulose, sodium carbomethylcellulose; and/or physiologically acceptable polymers such as polyvinylpyrrolidone (PVP). If desired, disintegrating agents may be added, such as cross-linked polyvinyl pyrrolidone, agar, or alginic acid or a salt thereof such as sodium alginate.

Dragee cores are provided with suitable coatings. For this purpose, concentrated sugar solutions may be used which may optionally contain gum arabic, talc, polyvinyl pyrrolidone, carbopol gel, polyethylene glycol, titanium dioxide, lacquer solutions and suitable organic solvents or solvent mixtures. Dyestuffs or pigments may be added to the tablets or dragee coatings for identification or to characterize different combinations of active compound doses.

Pharmaceutical compositions which can be used orally, include push-fit capsules made of gelatin as well as soft, sealed capsules made of gelatin and a plasticizer, such as glycerol or sorbitol. The push-fit capsules may contain the active ingredients in admixture with filler such as lactose, binders such as starches, lubricants such as talc or magnesium stearate and, optionally, stabilizers. In soft capsules, the active ingredients may be dissolved or suspended in suitable liquids, such as fatty oils, liquid paraffin, or liquid polyethylene glycols. In addition, stabilizers may be added. All formulations for oral administration should be in dosages suitable for the chosen route of administration.

For buccal administration, the compositions may take the form of tablets or lozenges formulated in conventional manner.

For administration by nasal inhalation, the active ingredients for use according to some embodiments of the invention are conveniently delivered in the form of an aerosol spray presentation from a pressurized pack or a nebulizer with the use of a suitable propellant, e.g., dichlorodifluoromethane, trichlorofluoromethane, dichloro-tetrafluoroethane or carbon dioxide. In the case of a pressurized aerosol, the dosage unit may be determined by providing a valve to deliver a metered amount. Capsules and cartridges of, e.g., gelatin for use in a dispenser may be formulated containing a powder mix of the compound and a suitable powder base such as lactose or starch.

The pharmaceutical composition described herein may be formulated for parenteral administration, e.g., by bolus injection or continuous infusion. Formulations for injection may be presented in unit dosage form, e.g., in ampoules or in multidose containers with optionally, an added preservative. The compositions may be suspensions, solutions or emulsions in oily or aqueous vehicles, and may contain formulator agents such as suspending, stabilizing and/or dispersing agents.

Pharmaceutical compositions for parenteral administration include aqueous solutions of the active preparation in water-soluble form. Additionally, suspensions of the active ingredients may be prepared as appropriate oily or water based injection suspensions. Suitable lipophilic solvents or vehicles include fatty oils such as sesame oil, or synthetic fatty acids esters such as ethyl oleate, triglycerides or liposomes. Aqueous injection suspensions may contain substances, which increase the viscosity of the suspension, such as sodium carboxymethyl cellulose, sorbitol or dextran. Optionally, the suspension may also contain suitable stabilizers or agents which increase the solubility of the active ingredients to allow for the preparation of highly concentrated solutions.

Alternatively, the active ingredient may be in powder form for constitution with a suitable vehicle, e.g., sterile, pyrogen-free water based solution, before use.

The pharmaceutical composition of some embodiments of the invention may also be formulated in rectal compositions such as suppositories or retention enemas, using, e.g., conventional suppository bases such as cocoa butter or other glycerides.

Pharmaceutical compositions suitable for use in context of some embodiments of the invention include compositions wherein the active ingredients are contained in an amount effective to achieve the intended purpose. More specifically, a therapeutically effective amount means an amount of active ingredients effective to prevent, alleviate or ameliorate symptoms of a disorder (e.g., cancer) or prolong the survival of the subject being treated.

Determination of a therapeutically effective amount is well within the capability of those skilled in the art, especially in light of the detailed disclosure provided herein.

For any preparation used in the methods of the invention, the therapeutically effective amount or dose can be estimated initially from in vitro and cell culture assays. For example, a dose can be formulated in animal models to achieve a desired concentration or titer. Such information can be used to more accurately determine useful doses in humans.

Toxicity and therapeutic efficacy of the active ingredients described herein can be determined by standard pharmaceutical procedures in vitro, in cell cultures or experimental animals. The data obtained from these in vitro and cell culture assays and animal studies can be used in formulating a range of dosage for use in human. The dosage may vary depending upon the dosage form employed and the route of administration utilized. The exact formulation, route of administration and dosage can be chosen by the individual physician in view of the patient's condition. (See e.g., Fingl, et al., 1975, in "The Pharmacological Basis of Therapeutics", Ch. 1 p.1).

Dosage amount and interval may be adjusted individually to provide levels of the active ingredient that are sufficient to induce or suppress the biological effect (minimal effective concentration, MEC). The MEC will vary for each preparation, but can be estimated from in vitro data. Dosages necessary to achieve the MEC will depend on individual characteristics and route of administration. Detection assays can be used to determine plasma concentrations.

Depending on the severity and responsiveness of the condition to be treated, dosing can be of a single or a plurality of administrations, with course of treatment lasting from several days to several weeks or until cure is effected or diminution of the disease state is achieved.

The amount of a composition to be administered will, of course, be dependent on the subject being treated, the severity of the affliction, the manner of administration, the judgment of the prescribing physician, etc.

Compositions of some embodiments of the invention may, if desired, be presented in a pack or dispenser device, such as an FDA approved kit, which may contain one or more unit dosage forms containing the active ingredient. The pack may, for example, comprise metal or plastic foil, such as a blister pack. The pack or dispenser device may be accompanied by instructions for administration. The pack or dispenser may also be accommodated by a notice associated with the container in a form prescribed by a governmental agency regulating the manufacture, use or sale of pharmaceuticals, which notice is reflective of approval by the agency of the form of the compositions or human or veterinary administration. Such notice, for example, may be of labeling approved by the U.S. Food and Drug Administration for prescription drugs or of an approved product insert. Compositions comprising a preparation of the invention formulated in a compatible pharmaceutical carrier may also be prepared, placed in an appropriate container, and labeled for treatment of an indicated condition, as is further detailed above.

The MEK and SRC inhibitor may be formulated in a single composition (co-formulation) or may each be comprised in two separate compositions.

In the context of a combination therapy, the MEK inhibitor may be administered by the same route of administration (e.g. intrapulmonary, oral, enteral, etc.) as the SRC inhibitor is administered. In the alternative, the MEK inhibitor may be administered by a different route of administration to the SRC inhibitor.

The MEK inhibitor can be administered immediately prior to (or after) the SRC inhibitor, on the same day as, one day before (or after), one week before (or after), one month before (or after), or two months before (or after) the SRC inhibitor, and the like.

The MEK inhibitor and the SRC inhibitor can be administered concomitantly, that is, where the administering for each of these reagents can occur at time intervals that partially or fully overlap each other. The MEK inhibitor and the SRC inhibitor can be administered during time intervals that do not overlap each other. For example, the MEK inhibitor can be administered within the time frame of t=0 to 1 hours, while the SRC inhibitor can be administered within the time frame of t=1 to 2 hours. Also, the MEK inhibitor can be administered within the time frame of t=0 to 1 hours, while the SRC inhibitor can be administered somewhere within the time frame of t=2-3 hours, t=3-4 hours, t=4-5 hours, t=5-6 hours, t=6-7 hours, t=7-8 hours, t=8-9 hours, t=9-10 hours, and the like. Moreover, the SRC inhibitor can be administered somewhere in the time frame of t=minus 2-3 hours, t=minus 3-4 hours, t=minus 4-5 hours, t=5-6 minus hours, t=minus 6-7 hours, t=minus 7-8 hours, t=minus 8-9 hours, t=minus 9-10 hours.

The SRC inhibitor of the present invention and the MEK inhibitor are typically provided in combined amounts to achieve therapeutic, prophylactic and/or pain palliative effectiveness. This amount will evidently depend upon the particular compound selected for use, the nature and number of the other treatment modality, the condition(s) to be treated, prevented and/or palliated, the species, age, sex, weight, health and prognosis of the subject, the mode of administration, effectiveness of targeting, residence time, mode of clearance, type and severity of side effects of the pharmaceutical composition and upon many other factors which will be evident to those of skill in the art. The SRC inhibitor will be used at a level at which therapeutic, prophylactic and/or pain palliating effectiveness in combination with the MEK inhibitor is observed.

The MEK inhibitor may be administered (together with the SRC inhibitor) at a gold standard dosing as a single agent, below a gold standard dosing as a single agent or above a gold standard dosing as a single agent.

According to specific embodiments, the MEK inhibitor is administered below the gold standard dosing as a single agent.

As used herein the term "gold standard dosing" refers to the dosing which is recommended by a regulatory agency (e.g., FDA), for a given tumor at a given stage.

According to other specific embodiments, the MEK inhibitor is administered at a dose that does not exert at least one side effect which is associated with the gold standard dosing.

Thus, in one embodiment, the amount of the MEK inhibitor is below the minimum dose required for therapeutic, prophylactic and/or pain palliative effectiveness when used as a single therapy (e.g. 10-99%, preferably 25 to 75% of that minimum dose). This allows for reduction of the side effects caused by the MEK inhibitor but the therapy is rendered effective because in combination with the SRC inhibitor, the combinations are effective overall.

In one aspect of the present invention, the SRC inhibitor and the MEK inhibitor are synergistic with respect to their dosages. That is to say that the effect provided by the SRC inhibitor of the present invention is greater than would be anticipated from the additive effects of the MEK inhibitor and the SRC inhibitor when used separately. In an alternative embodiment, the MEK inhibitor of the present invention and the SRC inhibitor are synergistic with respect to their side effects. That is to say that the side-effects caused by the SRC inhibitor in combination with the MEK inhibitor are less than would be anticipated when the equivalent therapeutic effect is provided by either the MEK inhibitor or SRC inhibitor when used separately.

Therapeutic regimen for treatment of cancer suitable for combination with the MEK and SRC inhibitors include, but are not limited to chemotherapy, radiotherapy, phototherapy and photodynamic therapy, surgery, nutritional therapy, ablative therapy, combined radiotherapy and chemotherapy, brachiotherapy, proton beam therapy, immunotherapy, cellular therapy and photon beam radiosurgical therapy.

Examples of anti-cancer drugs that can be co-administered (or even co-formulated) with the MEK and SRC inhibitors include, but are not limited to Acivicin; Aclarubicin; Acodazole Hydrochloride; Acronine; Adriamycin; Adozelesin; Aldesleukin; Altretamine; Ambomycin; Ametantrone Acetate; Aminoglutethimide; Amsacrine; Anastrozole; Anthramycin; Asparaginase; Asperlin; Azacitidine; Azetepa; Azotomycin; Batimastat; Benzodepa; Bicalutamide; Bisantrene Hydrochloride; Bisnafide Dimesylate; Bizelesin; Bleomycin Sulfate; Brequinar Sodium; Bropirimine; Busulfan; Cactinomycin; Calusterone; Caracemide; Carbetimer; Carboplatin; Carmustine; Carubicin Hydrochloride; Carzelesin; Cedefingol; Chlorambucil; Cirolemycin; Cisplatin; Cladribine; Crisnatol Mesylate; Cyclophosphamide; Cytarabine; Dacarbazine; Dactinomycin; Daunorubicin Hydrochloride; Decitabine; Dexormaplatin; Dezaguanine; Dezaguanine Mesylate; Diaziquone; Docetaxel; Doxorubicin; Doxorubicin Hydrochloride; Droloxifene; Droloxifene Citrate; Dromostanolone Propionate; Duazomycin; Edatrexate; Eflornithine Hydrochloride; Elsamitrucin; Enloplatin; Enpromate; Epipropidine; Epirubicin Hydrochloride; Erbulozole; Esorubicin Hydrochloride; Estramustine; Estramustine Phosphate Sodium; Etanidazole; Etoposide; Etoposide Phosphate; Etoprine; Fadrozole Hydrochloride; Fazarabine; Fenretinide; Floxuridine; Fludarabine Phosphate; Fluorouracil; Flurocitabine; Fosquidone; Fostriecin Sodium; Gemcitabine; Gemcitabine Hydrochloride; Hydroxyurea; Idarubicin Hydrochloride; Ifosfamide; Ilmofosine; Interferon Alfa-2a; Interferon Alfa-2b; Interferon Alfa-n1; Interferon Alfa-n3; Interferon Beta- I a; Interferon Gamma- I b; Iproplatin; Irinotecan Hydrochloride; Lanreotide Acetate; Letrozole; Leuprolide Acetate; Liarozole Hydrochloride; Lometrexol Sodium; Lomustine; Losoxantrone Hydrochloride; Masoprocol; Maytansine; Mechlorethamine Hydrochloride; Megestrol Acetate; Melengestrol Acetate; Melphalan; Menogaril; Mercaptopurine; Methotrexate; Methotrexate Sodium; Metoprine; Meturedepa; Mitindomide; Mitocarcin; Mitocromin; Mitogillin; Mitomalcin; Mitomycin; Mitosper; Mitotane; Mitoxantrone Hydrochloride; Mycophenolic Acid; Nocodazole; Nogalamycin; Ormaplatin; Oxisuran; Paclitaxel; Pegaspargase; Peliomycin; Pentamustine; Peplomycin Sulfate; Perfosfamide; Pipobroman; Piposulfan; Piroxantrone Hydrochloride; Plicamycin; Plomestane; Porfimer Sodium; Porfiromycin; Prednimustine; Procarbazine Hydrochloride; Puromycin; Puromycin Hydrochloride; Pyrazofurin; Riboprine; Rogletimide; Safingol; Safingol Hydrochloride; Semustine; Simtrazene; Sparfosate Sodium; Sparsomycin; Spirogermanium Hydrochloride; Spiromustine; Spiroplatin; Streptonigrin; Streptozocin; Sulofenur; Talisomycin; Taxol; Tecogalan Sodium; Tegafur; Teloxantrone Hydrochloride; Temoporfin; Teniposide; Teroxirone; Testolactone; Thiamiprine; Thioguanine; Thiotepa; Tiazofuirin; Tirapazamine; Topotecan Hydrochloride; Toremifene Citrate; Trestolone Acetate; Triciribine Phosphate; Trimetrexate; Trimetrexate Glucuronate; Triptorelin; Tubulozole Hydrochloride; Uracil Mustard; Uredepa; Vapreotide; Verteporfin; Vinblastine Sulfate; Vincristine Sulfate; Vindesine; Vindesine Sulfate; Vinepidine Sulfate; Vinglycinate Sulfate; Vinleurosine Sulfate; Vinorelbine Tartrate; Vinrosidine Sulfate; Vinzolidine Sulfate; Vorozole; Zeniplatin; Zinostatin; Zorubicin Hydrochloride. Additional antineoplastic agents include those disclosed in Chapter 52, Antineoplastic Agents (Paul Calabresi and Bruce A. Chabner), and the introduction thereto, 1202-1263, of Goodman and Gilman's "The Pharmacological Basis of Therapeutics", Eighth Edition, 1990, McGraw-Hill, Inc. (Health Professions Division).

According to a particular embodiment, the MEK and SRC inhibitor are provided together with Folfox (folinic acid, fluorouracil, and oxaliplatin) or Folfiri (leucovorin calcium (calcium folinate), 5-fluorouracil, and irinotecan).

According to another aspect of the present invention there is provided a method of selecting treatment to colorectal cancer (CRC) for a subject in need thereof, the method comprising determining a level of SRC p419 and a RAS mutation to exclude a KRAS G12 mutation in cancer cells of the subject, wherein a SRC p419 level above a predetermined level and an absence of a KRAS G12 mutation, is indicative of an efficacious treatment with of a MEK inhibitor and a SRC inhibitor.

Methods of analyzing cancer cells to ascertain the levels of SRC p419 and to exclude a KRAS G12 mutation are described herein above.

Once the subject has been selected, tissue retrieved therefrom may be further tested to analyze sensitivity of the treatments.

In one embodiment, the tissue is tested using an EVOC model.

The EVOC model uses a method which involves culturing a precision-cut tissue slice on a tissue culture insert in culture medium under a highly oxygenated atmosphere containing at least 50 % oxygen; and agitating the culture in a rotation facilitating intermittent submersion of the tissue slice in the culture medium
As used herein, the term "culture system" refers to at least a precision-cut tissue slice, insert and medium in an *ex-vivo* environment.

According to specific embodiments, the culture system maintains structure and viability of the precision-cut tissue slice for at least 2-10, 2-7, 2-5, 4-7, 5-7 or 4-5 days in culture. According to a specific embodiment, the precision-cut tissue slice maintains viability for at least 5 days, 6 days, 7 days or even 10 days. According to a specific embodiment, the precision-cut tissue slice maintains viability for at least 5 days.

According to specific embodiments, at least 60 %, at least 70 %, at least 80 % of the cells in the precision-cut tissue maintain viability following 4-5 days in culture as determined by e.g. morphology analysis of an optimal area of viability.

As used herein, the phrase "optimal area of viability" refers to a microscopic field of the tissue (e.g. in 20X magnification) in which the highest number of live cells per unit area are present, as assessed by a pathologist, in comparison to the immediate pre-EVOC sample of the same species.

Thus, according to specific embodiments, the culturing is effected for 2-10, 2-7, 2-5, 4-7, 5-7 or 4-5 days.

According to a specific embodiment, the culturing is effected for at least 4 days.

According to a specific embodiment, the culturing is effected for at least 5 days.

According to a specific embodiment, the culturing is effected for up to 7 days.
As used herein a "pharmaceutical composition" refers to a preparation of one or more of the active ingredients described herein with other chemical components such as physiologically suitable carriers and excipients. The purpose of a pharmaceutical composition is to facilitate administration of a compound to an organism.

Herein the term "active ingredient" refers to the agent that inhibits the synthesis and/or activity of cortisol which is accountable for the biological effect.

Hereinafter, the phrases "physiologically acceptable carrier" and "pharmaceutically acceptable carrier" which may be interchangeably used refer to a carrier or a diluent that does not cause significant irritation to an organism and does not abrogate the biological activity and properties of the administered compound. An adjuvant is included under these phrases.

Herein the term "excipient" refers to an inert substance added to a pharmaceutical composition to further facilitate administration of an active ingredient. Examples, without limitation, of excipients include calcium carbonate, calcium phosphate, various sugars and types of starch, cellulose derivatives, gelatin, vegetable oils and polyethylene glycols.

Techniques for formulation and administration of drugs may be found in "Remington's Pharmaceutical Sciences," Mack Publishing Co., Easton, PA, latest edition, which is incorporated herein by reference.

Suitable routes of administration may, for example, include oral, rectal, transmucosal, especially transnasal, intestinal or parenteral delivery, including intramuscular, subcutaneous and intramedullary injections as well as intrathecal, direct intraventricular, intracardiac, e.g., into the right or left ventricular cavity, into the common coronary artery, intravenous, intraperitoneal, intranasal, or intraocular injections.

Conventional approaches for drug delivery to the central nervous system (CNS) include: neurosurgical strategies (e.g., intracerebral injection or intracerebroventricular infusion); molecular manipulation of the agent (e.g., production of a chimeric fusion protein that comprises a transport peptide that has an affinity for an endothelial cell surface molecule in combination with an agent that is itself incapable of crossing the BBB) in an attempt to exploit one of the endogenous transport pathways of the BBB; pharmacological strategies designed to increase the lipid solubility of an agent (e.g., conjugation of water-soluble agents to lipid or cholesterol carriers); and the transitory disruption of the integrity of the BBB by hyperosmotic disruption (resulting from the infusion of a mannitol solution into the carotid artery or the use of a biologically active agent such as an angiotensin peptide). However, each of these strategies has limitations, such as the inherent risks associated with an invasive surgical procedure, a size limitation imposed by a limitation inherent in the endogenous transport systems, potentially undesirable biological side effects associated with the systemic administration of a chimeric molecule comprised of a carrier motif that could be active outside of the CNS, and the possible risk of brain damage within regions of the brain where the BBB is disrupted, which renders it a suboptimal delivery method.

Alternately, one may administer the pharmaceutical composition in a local rather than systemic manner, for example, via injection of the pharmaceutical composition directly into a tissue region of a patient.

The term "tissue" refers to part of an organism consisting of cells designed to perform a function or functions. Examples include, but are not limited to, brain tissue, retina, skin tissue, hepatic tissue, pancreatic tissue, bone, cartilage, connective tissue, blood tissue, muscle tissue, cardiac tissue brain tissue, vascular tissue, renal tissue, pulmonary tissue, gonadal tissue, hematopoietic tissue.

Pharmaceutical compositions of some embodiments of the invention may be manufactured by processes well known in the art, e.g., by means of conventional mixing, dissolving, granulating, dragee-making, levigating, emulsifying, encapsulating, entrapping or lyophilizing processes.

Pharmaceutical compositions for use in accordance with some embodiments of the invention thus may be formulated in conventional manner using one or more physiologically acceptable carriers comprising excipients and auxiliaries, which facilitate processing of the active ingredients into preparations which, can be used pharmaceutically. Proper formulation is dependent upon the route of administration chosen.

For injection, the active ingredients of the pharmaceutical composition may be formulated in aqueous solutions, preferably in physiologically compatible buffers such as Hank's solution, Ringer's solution, or physiological salt buffer. For transmucosal administration, penetrants appropriate to the barrier to be permeated are used in the formulation. Such penetrants are generally known in the art.

For oral administration, the pharmaceutical composition can be formulated readily by combining the active compounds with pharmaceutically acceptable carriers well known in the art. Such carriers enable the pharmaceutical composition to be formulated as tablets, pills, dragees, capsules, liquids, gels, syrups, slurries, suspensions, and the like, for oral ingestion by a patient. Pharmacological preparations for oral use can be made using a solid excipient, optionally grinding the resulting mixture, and processing the mixture of granules, after adding suitable auxiliaries if desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethyl-cellulose, sodium carbomethylcellulose; and/or physiologically acceptable polymers such as polyvinylpyrrolidone (PVP). If desired, disintegrating agents may be added, such as cross-linked polyvinyl pyrrolidone, agar, or alginic acid or a salt thereof such as sodium alginate.

Dragee cores are provided with suitable coatings. For this purpose, concentrated sugar solutions may be used which may optionally contain gum arabic, talc, polyvinyl pyrrolidone, carbopol gel, polyethylene glycol, titanium dioxide, lacquer solutions and suitable organic solvents or solvent mixtures. Dyestuffs or pigments may be added to the tablets or dragee coatings for identification or to characterize different combinations of active compound doses.

Pharmaceutical compositions which can be used orally, include push-fit capsules made of gelatin as well as soft, sealed capsules made of gelatin and a plasticizer, such as glycerol or sorbitol. The push-fit capsules may contain the active ingredients in admixture with filler such as lactose, binders such as starches, lubricants such as talc or magnesium stearate and, optionally, stabilizers. In soft capsules, the active ingredients may be dissolved or suspended in suitable liquids, such as fatty oils, liquid paraffin, or liquid polyethylene glycols. In addition, stabilizers may be added. All formulations for oral administration should be in dosages suitable for the chosen route of administration.

For buccal administration, the compositions may take the form of tablets or lozenges formulated in conventional manner.

For administration by nasal inhalation, the active ingredients for use according to some embodiments of the invention are conveniently delivered in the form of an aerosol spray presentation from a pressurized pack or a nebulizer with the use of a suitable propellant, e.g., dichlorodifluoromethane, trichlorofluoromethane, dichloro-tetrafluoroethane or carbon dioxide. In the case of a pressurized aerosol, the dosage unit may be determined by providing a valve to deliver a metered amount. Capsules and cartridges of, e.g., gelatin for use in a dispenser may be formulated containing a powder mix of the compound and a suitable powder base such as lactose or starch.

The pharmaceutical composition described herein may be formulated for parenteral administration, e.g., by bolus injection or continuous infusion. Formulations for injection may be presented in unit dosage form, e.g., in ampoules or in multidose containers with optionally, an added preservative. The compositions may be suspensions, solutions or emulsions in oily or aqueous vehicles, and may contain formulatory agents such as suspending, stabilizing and/or dispersing agents.

Pharmaceutical compositions for parenteral administration include aqueous solutions of the active preparation in water-soluble form. Additionally, suspensions of the active ingredients may be prepared as appropriate oily or water based injection suspensions. Suitable lipophilic solvents or vehicles include fatty oils such as sesame oil, or synthetic fatty acids esters such as ethyl oleate, triglycerides or liposomes. Aqueous injection suspensions may contain substances, which increase the viscosity of the suspension, such as sodium carboxymethyl cellulose, sorbitol or dextran. Optionally, the suspension may also contain suitable stabilizers or agents which increase the solubility of the active ingredients to allow for the preparation of highly concentrated solutions.

Alternatively, the active ingredient may be in powder form for constitution with a suitable vehicle, e.g., sterile, pyrogen-free water based solution, before use.

The pharmaceutical composition of some embodiments of the invention may also be formulated in rectal compositions such as suppositories or retention enemas, using, e.g., conventional suppository bases such as cocoa butter or other glycerides.

Pharmaceutical compositions suitable for use in context of some embodiments of the invention include compositions wherein the active ingredients are contained in an amount effective to achieve the intended purpose. More specifically, a therapeutically effective amount means an amount of active ingredients effective to prevent, alleviate or ameliorate symptoms of a disorder (e.g., cancer) or prolong the survival of the subject being treated.

Determination of a therapeutically effective amount is well within the capability of those skilled in the art, especially in light of the detailed disclosure provided herein.

For any preparation used in the methods of the invention, the therapeutically effective amount or dose can be estimated initially from in vitro and cell culture assays. For example, a dose can be formulated in animal models to achieve a desired concentration or titer. Such information can be used to more accurately determine useful doses in humans.

Toxicity and therapeutic efficacy of the active ingredients described herein can be determined by standard pharmaceutical procedures in vitro, in cell cultures or experimental animals. The data obtained from these in vitro and cell culture assays and animal studies can be used in formulating a range of dosage for use in human. The dosage may vary depending upon the dosage form employed and the route of administration utilized. The exact formulation, route of administration and dosage can be chosen by the individual physician in view of the patient's condition. (See e.g., Fingl, et al., 1975, in "The Pharmacological Basis of Therapeutics", Ch. 1 p.1).

Dosage amount and interval may be adjusted individually to provide levels of the active ingredient that are sufficient to induce or suppress the biological effect (minimal effective concentration, MEC). The MEC will vary for each preparation, but can be estimated from in vitro data. Dosages necessary to achieve the MEC will depend on individual characteristics and route of administration. Detection assays can be used to determine plasma concentrations.

Depending on the severity and responsiveness of the condition to be treated, dosing can be of a single or a plurality of administrations, with course of treatment lasting from several days to several weeks or until cure is effected or diminution of the disease state is achieved.

The amount of a composition to be administered will, of course, be dependent on the subject being treated, the severity of the affliction, the manner of administration, the judgment of the prescribing physician, etc.

Compositions of some embodiments of the invention may, if desired, be presented in a pack or dispenser device, such as an FDA approved kit, which may contain one or more unit dosage forms containing the active ingredient. The pack may, for example, comprise metal or plastic foil, such as a blister pack. The pack or dispenser device may be accompanied by instructions for administration. The pack or dispenser may also be accommodated by a notice associated with the container in a form prescribed by a governmental agency regulating the manufacture, use or sale of pharmaceuticals, which notice is reflective of approval by the agency of the form of the compositions or human or veterinary administration. Such notice, for example, may be of labeling approved by the U.S. Food and Drug Administration for prescription drugs or of an approved product insert. Compositions comprising a preparation of the invention formulated in a compatible pharmaceutical carrier may also be prepared, placed in an appropriate container, and labeled for treatment of an indicated condition, as is further detailed above.

The MEK and SRC inhibitor may be formulated in a single composition (co-formulation) or may each be comprised in two separate compositions.

In the context of a combination therapy, the MEK inhibitor may be administered by the same route of administration (e.g. intrapulmonary, oral, enteral, etc.) as the SRC inhibitor is administered. In the alternative, the MEK inhibitor may be administered by a different route of administration to the SRC inhibitor.

The MEK inhibitor can be administered immediately prior to (or after) the SRC inhibitor, on the same day as, one day before (or after), one week before (or after), one month before (or after), or two months before (or after) the SRC inhibitor, and the like.

The MEK inhibitor and the SRC inhibitor can be administered concomitantly, that is, where the administering for each of these reagents can occur at time intervals that partially or fully overlap each other. The MEK inhibitor and the SRC inhibitor can be administered during time intervals that do not overlap each other. For example, the MEK inhibitor can be administered within the time frame of t=0 to 1 hours, while the SRC inhibitor can be administered within the time frame of t=1 to 2 hours. Also, the MEK inhibitor can be administered within the time frame of t=0 to 1 hours, while the SRC inhibitor can be administered somewhere within the time frame of t=2-3 hours, t=3-4 hours, t=4-5 hours, t=5-6 hours, t=6-7 hours, t=7-8 hours, t=8-9 hours, t=9-10 hours, and the like. Moreover, the SRC inhibitor can be administered somewhere in the time frame of t=minus 2-3 hours, t=minus 3-4 hours, t=minus 4-5 hours, t=5-6 minus hours, t=minus 6-7 hours, t=minus 7-8 hours, t=minus 8-9 hours, t=minus 9-10 hours.

The SRC inhibitor of the present invention and the MEK inhibitor are typically provided in combined amounts to achieve therapeutic, prophylactic and/or pain palliative effectiveness. This amount will evidently depend upon the particular compound selected for use, the nature and number of the other treatment modality, the condition(s) to be treated, prevented and/or palliated, the species, age, sex, weight, health and prognosis of the subject, the mode of administration, effectiveness of targeting, residence time, mode of clearance, type and severity of side effects of the pharmaceutical composition and upon many other factors which will be evident to those of skill in the art. The SRC inhibitor will be used at a level at which therapeutic, prophylactic and/or pain palliating effectiveness in combination with the MEK inhibitor is observed.

The MEK inhibitor may be administered (together with the SRC inhibitor) at a gold standard dosing as a single agent, below a gold standard dosing as a single agent or above a gold standard dosing as a single agent.

According to specific embodiments, the MEK inhibitor is administered below the gold standard dosing as a single agent.

As used herein the term "gold standard dosing" refers to the dosing which is recommended by a regulatory agency (e.g., FDA), for a given tumor at a given stage.

According to other specific embodiments, the MEK inhibitor is administered at a dose that does not exert at least one side effect which is associated with the gold standard dosing.

Thus, in one embodiment, the amount of the MEK inhibitor is below the minimum dose required for therapeutic, prophylactic and/or pain palliative effectiveness when used as a single therapy (e.g. 10-99%, preferably 25 to 75% of that minimum dose). This allows for reduction of the side effects caused by the MEK inhibitor but the therapy is rendered effective because in combination with the SRC inhibitor, the combinations are effective overall.

In one aspect of the present invention, the SRC inhibitor and the MEK inhibitor are synergistic with respect to their dosages. That is to say that the effect provided by the SRC inhibitor of the present invention is greater than would be anticipated from the additive effects of the MEK inhibitor and the SRC inhibitor when used separately. In an alternative embodiment, the MEK inhibitor of the present invention and the SRC inhibitor are synergistic with respect to their side effects. That is to say that the side-effects caused by the SRC inhibitor in combination with the MEK inhibitor are less than would be anticipated when the equivalent therapeutic effect is provided by either the MEK inhibitor or SRC inhibitor when used separately.

Therapeutic regimen for treatment of cancer suitable for combination with the MEK and SRC inhibitors include, but are not limited to chemotherapy, radiotherapy, phototherapy and photodynamic therapy, surgery, nutritional therapy, ablative therapy, combined radiotherapy and chemotherapy, brachiotherapy, proton beam therapy, immunotherapy, cellular therapy and photon beam radiosurgical therapy.

Examples of anti-cancer drugs that can be co-administered (or even co-formulated) with the MEK and SRC inhibitors include, but are not limited to Acivicin; Aclarubicin; Acodazole Hydrochloride; Acronine; Adriamycin; Adozelesin; Aldesleukin; Altretamine; Ambomycin; Ametantrone Acetate; Aminoglutethimide; Amsacrine; Anastrozole; Anthramycin; Asparaginase; Asperlin; Azacitidine; Azetepa; Azotomycin; Batimastat; Benzodepa; Bicalutamide; Bisantrene Hydrochloride; Bisnafide Dimesylate; Bizelesin; Bleomycin Sulfate; Brequinar Sodium; Bropirimine; Busulfan; Cactinomycin; Calusterone; Caracemide; Carbetimer; Carboplatin; Carmustine; Carubicin Hydrochloride; Carzelesin; Cedefingol; Chlorambucil; Cirolemycin; Cisplatin; Cladribine; Crisnatol Mesylate; Cyclophosphamide; Cytarabine; Dacarbazine; Dactinomycin; Daunorubicin Hydrochloride; Decitabine; Dexormaplatin; Dezaguanine; Dezaguanine Mesylate; Diaziquone; Docetaxel; Doxorubicin; Doxorubicin Hydrochloride; Droloxifene; Droloxifene Citrate; Dromostanolone Propionate; Duazomycin; Edatrexate; Eflornithine Hydrochloride; Elsamitrucin; Enloplatin; Enpromate; Epipropidine; Epirubicin Hydrochloride; Erbulozole; Esorubicin Hydrochloride; Estramustine; Estramustine Phosphate Sodium; Etanidazole; Etoposide; Etoposide Phosphate; Etoprine; Fadrozole Hydrochloride; Fazarabine; Fenretinide; Floxuridine; Fludarabine Phosphate; Fluorouracil; Flurocitabine; Fosquidone; Fostriecin Sodium; Gemcitabine; Gemcitabine Hydrochloride; Hydroxyurea; Idarubicin Hydrochloride; Ifosfamide; Ilmofosine; Interferon Alfa-2a; Interferon Alfa-2b; Interferon Alfa-n1; Interferon Alfa-n3; Interferon Beta- I a; Interferon Gamma- I b; Iproplatin; Irinotecan Hydrochloride; Lanreotide Acetate; Letrozole; Leuprolide Acetate; Liarozole Hydrochloride; Lometrexol Sodium; Lomustine; Losoxantrone Hydrochloride; Masoprocol; Maytansine; Mechlorethamine Hydrochloride; Megestrol Acetate; Melengestrol Acetate; Melphalan; Menogaril; Mercaptopurine; Methotrexate; Methotrexate Sodium; Metoprine; Meturedepa; Mitindomide; Mitocarcin; Mitocromin; Mitogillin; Mitomalcin; Mitomycin; Mitosper; Mitotane; Mitoxantrone Hydrochloride; Mycophenolic Acid; Nocodazole; Nogalamycin; Ormaplatin; Oxisuran; Paclitaxel; Pegaspargase; Peliomycin; Pentamustine; Peplomycin Sulfate; Perfosfamide; Pipobroman; Piposulfan; Piroxantrone Hydrochloride; Plicamycin; Plomestane; Porfimer Sodium; Porfiromycin; Prednimustine; Procarbazine Hydrochloride; Puromycin; Puromycin Hydrochloride; Pyrazofurin; Riboprine; Rogletimide; Safingol; Safingol Hydrochloride; Semustine; Simtrazene; Sparfosate Sodium; Sparsomycin; Spirogermanium Hydrochloride; Spiromustine; Spiroplatin; Streptonigrin; Streptozocin; Sulofenur; Talisomycin; Taxol; Tecogalan Sodium; Tegafur; Teloxantrone Hydrochloride; Temoporfin; Teniposide; Teroxirone; Testolactone; Thiamiprine; Thioguanine; Thiotepa; Tiazofuirin; Tirapazamine; Topotecan Hydrochloride; Toremifene Citrate; Trestolone Acetate; Triciribine Phosphate; Trimetrexate; Trimetrexate Glucuronate; Triptorelin; Tubulozole Hydrochloride; Uracil Mustard; Uredepa; Vapreotide; Verteporfin; Vinblastine Sulfate; Vincristine Sulfate; Vindesine; Vindesine Sulfate; Vinepidine Sulfate; Vinglycinate Sulfate; Vinleurosine Sulfate; Vinorelbine Tartrate; Vinrosidine Sulfate; Vinzolidine Sulfate; Vorozole; Zeniplatin; Zinostatin; Zorubicin Hydrochloride. Additional antineoplastic agents include those disclosed in Chapter 52, Antineoplastic Agents (Paul Calabresi and Bruce A. Chabner), and the introduction thereto, 1202-1263, of Goodman and Gilman's "The Pharmacological Basis of Therapeutics", Eighth Edition, 1990, McGraw-Hill, Inc. (Health Professions Division).

According to a particular embodiment, the MEK and SRC inhibitor are provided together with Folfox (folinic acid, fluorouracil, and oxaliplatin) or Folfiri (leucovorin calcium (calcium folinate), 5-fluorouracil, and irinotecan).

According to another aspect of the present invention there is provided a method of selecting treatment to colorectal cancer (CRC) for a subject in need thereof, the method comprising determining a level of SRC p419 and a RAS mutation to exclude a KRAS G12 mutation in cancer cells of the subject, wherein a SRC p419 level above a predetermined level and an absence of a KRAS G12 mutation, is indicative of an efficacious treatment with of a MEK inhibitor and a SRC inhibitor.

Once the subject has been selected, tissue retrieved therefrom may be further tested to analyze sensitivity of the treatment.

Methods of determining effects induced by the drug or the drug combination are known in the art and include for example:
- Viability evaluation using e.g. the MTT test which is based on the selective ability of living cells to reduce the yellow salt MTT (3-(4, 5- dimethylthiazolyl-2)-2, 5-diphenyltetrazolium bromide) (Sigma, Aldrich St Louis, MO, USA) to a purple-blue insoluble formazan precipitate; the WST assay or the ATP uptake assay;
- Proliferation evaluation using e.g. the BrDu assay [Cell Proliferation ELISA BrdU colorimetric kit (Roche, Mannheim, Germany] or Ki67 staining;
- Cell death evaluation using e.g. the TUNEL assay [Roche, Mannheim, Germany] the Annexin V assay [ApoAlert^{®} Annexin V Apoptosis Kit (Clontech Laboratories, Inc., CA, USA)], the LDH assay, the Activated Caspase 3 assay, the Activated Caspase 8 assay and the Nitric Oxide Synthase assay;
- Senescence evaluation using e.g. the Senescence associated-β-galactosidase assay (Dimri GP, Lee X, et al. 1995. A biomarker that identifies senescent human cells in culture and in aging skin in vivo. Proc Natl Acad Sci U S A 92:9363-9367) and telomerase shortening assay;
- Cell metabolism evaluation using e.g. the glucose uptake assay;
- Various RNA and protein detection methods (which detect level of expression and/or activity); and
- Morphology evaluation using e.g. the Haemaotxylin & Eosin (H&E) staining;

According to specific embodiments, the determining is effected by morphology evaluation, viability evaluation, proliferation evaluation and/or cell death evaluation.

According to specific embodiments, the determining is effected by morphology evaluation.

Morphology evaluation using H&E staining can provide details on e.g. cell content, size and density, ratio of viable cells/dead cells, ratio of diseased (e.g. tumor) cells/healthy cells, immune cells infiltration, fibrosis, nuclear size and density and integrity, apoptotic bodies and mitotic figures.

Typically, results from each of the assays are expressed in a numeric form wherein a high score correlates with drug sensitivity and a low score correlated with drug resistance.

As used herein the phrase "sensitivity to a drug" or "sensitivity to drug combination" refers to the ability of a drug or drug combination to induce cellular changes such as changes in cell viability, proliferation rate, differentiation, cell death, necrosis, apoptosis, senescence, transcription and/or translation rate of specific genes and/or changes in protein states e.g. phosphorylation, dephosphorylation, translocation and any combinations thereof. According to specific embodiments, the cellular changes are reflected by decreased cell viability, decreased proliferation rate, increased cell death and/or aberrant morphology as compared to same in the absence of the drug.

According to a specific embodiment the cellular changes are reflected by decreased cell viability.

According to a specific embodiment the cellular changes are reflected by aberrant morphology.

According to specific embodiments the change is at least 1.5 fold, at least 2 fold, at least 3 fold, at least 5 fold, at least 10 fold, or at least 20 fold as compared to same in the absence of the drug.

According to other specific embodiments the change is by at least 5 %, by at least a 10 %, at least 20 %, at least 30 %, at least 40 %, at least 50 %, at least 60 %, at least 70 %, at least 80 %, at least 90 %, at least 95 %, at least 99 % or at least 100 % as compared to same in the absence of the drug.

According to specific embodiments, the determined efficacy of the drug indicates the potency of a batch of a drug, that is, drug sensitivity is indicative that the batch is potent.

As used herein, the term "potency" refers to the measure of the biological activity of the drug, based on the attribute of the drug which is linked to the relevant biological properties (i.e.; drug sensitivity).

According to specific embodiments of the invention, the method comprising comparing the sensitivity of the tissue to the drug with sensitivity of the tissue to a reference standard batch of the drug, so as to determine the relative potency of the batch.

As used herein, the term "relative potency" refers to a qualitative measure of potency of a batch of the drug, relatively to a standard reference (RS) of the drug, having a known potency.

According to specific embodiments the potency of a batch of the drug, is determined relatively to the known potency of a reference standard (RS).

As used herein, the phrase "reference standard" or "RS" refers to a standardized drug, which is used as a measurement base for the drug. RS provides a calibrated level of biological effect against which new preparations of the drug can be compared to.

According to a specific embodiment, the RS is characterized by optimum potency and quality of an active component that is effective in treating the disease (e.g., inflammatory disease, cancer).

Calculating potency and relative potency are known in the art. According to specific embodiments the relative potency is calculated using a software suitable for biological assays, such as parallel line analysis software e.g., PLA (Stegmann Systems GmbH) and Gen5 data analysis software (BioTek).

Implementation of the method and/or system of embodiments of the invention can involve performing or completing selected tasks manually, automatically, or a combination thereof. Moreover, according to actual instrumentation and equipment of embodiments of the method and/or system of the invention, several selected tasks could be implemented by hardware, by software or by firmware or by a combination thereof using an operating system.

According to specific embodiments, the determined efficacy of the drug or the drug combination indicates suitability of the drug for the treatment of a disease.

In one embodiment, the tissue is tested using an EVOC model.

The EVOC model uses a method which involves culturing a precision-cut tissue slice on a tissue culture insert in culture medium under a highly oxygenated atmosphere containing at least 50 % oxygen; and agitating the culture in a rotation facilitating intermittent submersion of the tissue slice in the culture medium.

As used herein, the term "culture system" refers to at least a precision-cut tissue slice, insert and medium in an *ex-vivo* environment.

According to specific embodiments, the culture system maintains structure and viability of the precision-cut tissue slice for at least 2-10, 2-7, 2-5, 4-7, 5-7 or 4-5 days in culture. According to a specific embodiment, the precision-cut tissue slice maintains viability for at least 5 days, 6 days, 7 days or even 10 days. According to a specific embodiment, the precision-cut tissue slice maintains viability for at least 5 days.

According to specific embodiments, at least 60 %, at least 70 %, at least 80 % of the cells in the precision-cut tissue maintain viability following 4-5 days in culture as determined by e.g. morphology analysis of an optimal area of viability.

As used herein, the phrase "optimal area of viability" refers to a microscopic field of the tissue (e.g. in 20X magnification) in which the highest number of live cells per unit area are present, as assessed by a pathologist, in comparison to the immediate pre-EVOC sample of the same species.

Thus, according to specific embodiments, the culturing is effected for 2-10, 2-7, 2-5, 4-7, 5-7 or 4-5 days.

According to a specific embodiment, the culturing is effected for at least 4 days.

According to a specific embodiment, the culturing is effected for at least 5 days.

According to a specific embodiment, the culturing is effected for up to 7 days.

The tissue may be cut and cultured directly following tissue extraction (i.e. primary tissue) or following implantation in an animal model [i.e. a patient-derived xenograft (PDX)], each possibility represents a separate embodiment of the present invention.

The tissue or the tissue slice to some embodiments of the present invention can be freshly isolated or stored e.g., at 4 ° C or cryopreserved (i.e. frozen) at e.g. liquid nitrogen.

According to specific embodiments, the tissue or the tissue slice is freshly isolated (i.e., not more than 24 hours after retrieval from the subject and not subjected to preservation processes), as further disclosed hereinbelow.

According to specific embodiments, the tissue is cryopreserved following tissue retrieval and prior to cutting.

According to specific embodiments, the tissue is thawed prior to cutting.

According to specific embodiments, the tissue slice is cryopreserved following cutting.

According to specific embodiments, the tissue slice is thawed prior to culturing.

According to specific embodiments, the tissue is preserved at 4 °C in e.g. medium following tissue retrieval and prior to cutting.

According to specific embodiments the tissue slice is preserved at 4 °C in e.g. medium following cutting and prior to culturing.

According to specific embodiments, the preservation at 4 °C is effected for up to 120 hours, up to 96 hours, up to 72 hours or up to 48 hours.

According to specific embodiments, the preservation at 4 °C is effected for 24-48 hours.

Thus, according to specific embodiments, the method further comprises obtaining the tissue from the subject or from the animal model comprising the tissue.

As used herein the phrase "patient-derived xenograft (PDX)" refers to tissue generated by the implantation of a primary tissue into an animal from a different species relative to the donor of the primary tissue. According to specific embodiments the PDX is a tissue generated by implantation of a human primary tissue (e.g. cancerous tissue) into an immunodeficient mouse.

Following tissue extraction the tissue is sliced to precision-cut slices.

As used herein, the phrase "precision-cut tissue slice" refers to a viable slice obtained from an isolated solid tissue with reproducible, well defined thickness (e.g. ± 5 % variation in thickness between slices).

Typically, the tissue slice is a mini-model of the tissue which contains the cells of the tissue in their natural environment and retains the three-dimensional connectivity such as intercellular and cell-matrix interactions of the intact tissue with no selection of a particular cell type among the different cell type that constitutes the tissue or the organ. Precision-cutting reduces sources of error due to variations in slice thickness and damage to cut surfaces, which both contribute to uneven gas and nutrient exchange throughout tissue slices; it enhances reproducibility; and allows adjacent slices to be evaluated for histology and compared pair-wise under different experimental conditions.

The slice section can be cut in different orientations (e.g. anterior-posterior, dorsal-ventral, or nasal-temporal) and thickness. The size/thickness of the tissue section is based on the tissue source and the method used for sectioning. According to specific embodiment the thickness of the precision-cut slice allows maintaining tissue structure in culture.

According to specific embodiments the thickness of the precision-cut slice allows full access of the inner cell layers to oxygen and nutrients, such that the inner cell layers are exposed to sufficient oxygen and nutrients concentrations.

According to specific embodiments the thickness of the precision-cut slice allows full access of the inner cell layers to oxygen and nutrients, such that the inner cell layers are exposed to the same oxygen and nutrients concentrations as the outer cell layers.

According to specific embodiments, the precision-cut slice is between 50-1200 µm, between 100-1000 µm, between 100-500 µm, between 100-300 µm, or between 200-300 µm.

According to a specific embodiment, the precision-cut slice is 200-300 µm.

Methods of obtaining tissue slices are known in the art and described for examples in the Examples section which follows and in Roife et al. (2016) Clin. Cancer Res. June 3, 1-10; Vickers et al. (2004) Toxicol Sci. 82(2):534-44; Zimmermann et al. (2009) Cytotechnology 61(3): 145-152); Koch et al. (2014) Cell Communication and Signaling 12:73; and Graaf et al. Nature Protocols (2010) 5: 1540-1551, the contents of each of which are fully incorporated herein by reference. Such methods include, but are not limited to slicing using a vibratome, agarose embedding followed by sectioning by a microtome, or slicing using a matrix.

As a non-limiting example, the tissue is isolated and immediately placed in a physiological dissection media (e.g. ice cold PBS) which may be supplemented with antibiotics.

According to specific embodiments, the warm ischemic time is less than 2 hours, less than 1.5 hours or less than 1 hour.

According to specific embodiments, the cold ischemic time is less than 96 hours, less than 72 hours, less than 48 hours, less than 24 hours, less than 12 hour, less than 5 hours or less than 2 hours.

Prior to slicing, the tissue is attached to the tissue slicer using e.g. contact glue followed by embedding in e.g. low melting agarose gel. Subsequently, the tissue is sectioned into precision-cut slices. Numerous suitable tissue sectioning devices are commercially available, such as, but not limited to Compresstome^{™} VF-300 (Precisionary Instruments Inc. NC, USA), Brendel-Vitron tissue slicer (Tucson, AZ), Krumdieck precision tissue slicer (model no. MD4000-01; Alabama R&D) and Leica VT1200S vibrating blade microtome (Leica, Wetzlar, Germany). According to specific embodiments, the sectioning devise is filled with ice cold medium such as Williams Medium E or Krebs-Henseleit buffer (KHB). The skilled artisan would know which medium and conditions for dissection and for preserving the tissue and the tissue slice prior to culturing for each type of tissue.

Following, the tissue slice is placed on a tissue culture insert in a tissue culture vessel filled with culture medium. One slice or multiple slices can be placed on a single tissue culture insert. According to specific embodiments, one slice is placed on a single tissue culture insert.

According to specific embodiments, the culture vessel is filled with culture medium up to the bottom of the tissue slice (e.g. 4 ml of medium in a 6-well plate containing an insert).

The culture may be in a glass, plastic or metal vessel that can provide an aseptic environment for tissue culturing. According to specific embodiments, the culture vessel includes dishes, plates, flasks, bottles and vials. Culture vessels such as COSTAR^{®}, NUNC^{®} and FALCON^{®} are commercially available from various manufacturers.

According to specific embodiments, the culture vessel is a tissue culture plate such as a 6-wells plate, 24-wells plate, 48-wells plate and 96-wells plate.

According to a specific embodiment, the culture vessel is a tissue culture 6-wells plate.

According to specific embodiments, the culture vessel is not pre-coated with proteins extracted from a matched tissue (e.g. when the tissue is a cancerous tissue, then the culture vessel is not pre-coated with stage and grade-matched tumor extracted proteins). Non limiting examples for such proteins include ECM proteins such as collagen, fibronectin, laminin, vibronectin, cadherin, filamin A, vimentin, osteopontin, Decorin, tenascin X, basement membrane proteins, cytoskeletal proteins and matrix proteins; and growth factors.

The culture medium used by the present invention can be a water-based medium which includes a combination of substances such as salts, nutrients, minerals, vitamins, amino acids, nucleic acids and/or proteins such as cytokines, growth factors and hormones, all of which are needed for cell proliferation and are capable of maintaining structure and viability of the tissue. For example, a culture medium can be a synthetic tissue culture medium such as DMEM/F12 (can be obtained from e.g. Biological Industries), M199 (can be obtained from e.g. Biological Industries), RPMI (can be obtained from e.g. Gibco-Invitrogen Corporation products), M199 (can be obtained from e.g. Sigma-Aldrich), Ko-DMEM (can be obtained from e.g. Gibco-Invitrogen Corporation products), supplemented with the necessary additives as is further described hereinunder. Preferably, all ingredients included in the culture medium of the present invention are substantially pure, with a tissue culture grade.

The skilled artisan would know to select the culture medium for each type of tissue contemplated.

According to specific embodiments of the invention, the culture medium comprises serum e.g. fetal calf serum (FCS, can be obtained e.g. from Gibco-Invitrogen Corporation products).

According to specific embodiments, the culture medium comprises less than 10% serum.

According to specific embodiments, the culture medium comprises less than 2% serum obtained from the same species as the cultured tissue.

According to specific embodiments, the culture medium is devoid of serum obtained from the same species as the cultured tissue.

According to specific embodiments, the culture medium comprises less than 2% serum autologous to the cultured tissue (i.e. from the same subject).

According to specific embodiments, the culture medium is devoid of serum autologous to the cultured tissue (i.e. from the same subject).

According to specific embodiments, the culture medium comprises less than 2% human serum.

According to some embodiments of the invention, the culture medium is devoid of human serum.

According to some embodiments of the invention, the culture medium is devoid of any animal contaminants, *i.e.,* animal cells, fluid or pathogens (e.g., viruses infecting animal cells), i.e., being xeno-free.

According to some embodiments of the invention, the culture medium can further include antibiotics (e.g., penicillin, streptomycin, gentamycin), anti-fungal agents (e.g. amphotericin B), L-glutamine or NEAA (non-essential amino acids).

According to a specific embodiment, the medium comprises serum and antibiotics.

According to a specific embodiment, the medium comprises DMEM/F12, 5 % FCS, glutamine, penicillin, streptomycin, gentamycin and amphotericin B

It should be noted that the culture medium may be periodically refreshed to maintain sufficient levels of supplements and to remove metabolic waste products that can damage the tissue. According to specific embodiments, the culture medium is refreshed every 12-72 hours, every 24-72 hours, every 24-48 hours or every 12-48 hours.

According to specific embodiments, the culture medium is refreshed every 12-48 hours.

According to a specific embodiment, the culture medium is refreshed once after 12-24 hours and then every about 48 hours.

As used herein, the phrase "tissue culture insert" refers to a porous membrane suspended in a vessel for tissue culture and is compatible with subsequent *ex-vivo* culturing of a tissue slice. The pore size is capable of supporting the tissue slice while it is permeable to the culture medium enabling the passage of nutrients and metabolic waste to and from the slice, respectively. According to specific embodiments, the tissue slice is placed on the tissue culture insert, thereby allowing access of the culture medium to both the apical and basal surfaces of the tissue slice.

According to specific embodiments the pore size is 0.1 µm - 20 µm, 0.1 µm - 15 µm, 0.1 µm - 10 µm, 0.1 µm - 5 µm, 0.4 µm - 20 µm, 0.4 µm - 10 µm or 0.4 µm - 5 µm.

According to specific embodiments the pore size is 0.4 mm - 4 mm, 0.4 mm - 1 mm, 1 mm - 4 mm, 1 mm - 3 mm or 1 mm - 2 mm.

According to specific embodiments, the tissue culture insert is sterile.

According to specific embodiments, the tissue culture inset is disposable.

According to specific embodiments, the cell culture insert is re-usable and autoclavable.

The cell culture insert may be synthetic or natural, it can be inorganic or polymeric e.g. titanium, alumina, Polytetrafluoroethylene (PTFE), Teflon, stainless steel, polycarbonate, nitrocellulose and cellulose esters. According to specific embodiments, the cell culture insert is a titanium insert. Cell culture inserts that can be used with specific embodiments of the invention are commercially available from e.g. Alabama R&D, Millipore Corporation, Costar, Corning Incorporated, Nunc, **Vitron Inc.** and SEFAR and include, but not limited to MA0036 Well plate Inserts, BIOCOAT^{™}, Transwell^{®}, Millicell^{®}, Falcon^{®}-Cyclopore, Nunc^{®} Anapore, titanium-screen and Teflon-screen.

According to specific embodiments, the tissue culture insert is a titanium grid insert, such as but not limited to Titanium MA0036 Well plate Inserts (Alabama R&D).

According to specific embodiments, the tissue culture insert is not coated with an organic material such as collagen, fibronectin or polyethylene glycol (PEG).

According to specific embodiments, the tissue culture insert is not coated with proteins extracted from a matched tissue (e.g. when the tissue is a cancerous tissue, then the tissue culture insert is not pre-coated with stage and grade-matched tumor extracted proteins).

According to specific embodiments, the tissue slice is in direct contact with the tissue culture insert.

According to specific embodiments, the tissue slice is not separated from the culture medium by a heterologous organic material such as collagen, fibronectin or synthetic polymer (which is not part of the culture vessel) e.g., polyethylene glycol (PEG).

According to specific embodiments, the tissue slice is in direct contact with the culture medium.

According to specific embodiments, the tissue slice is placed in the middle of the cell culture insert. Thus, for example, when a titanium grid such as the Titanium MA0036 Well plate Inserts is applied the tissue slice is placed in the concavity located at the middle of the insert.

Following, the tissue slice is cultured (or maintained) at a physiological temperature, e.g. 37 °C., in a highly oxygenated humidified atmosphere containing at least 50 % oxygen and e.g. 5 % CO₂.

According to specific embodiments the highly oxygenated atmosphere contains at least 60 %, at least 70 % or at least 80 % oxygen.

According to specific embodiments, the highly oxygenated atmosphere contains at least 70 % oxygen.

According to other specific embodiments, the highly oxygenated atmosphere contains less than 95 % oxygen.

According to a specific embodiment, the highly oxygenated atmosphere contains about 80 % oxygen.

According to a specific embodiment, during the culturing process, the culture is agitated in a rotation facilitating intermittent submersion of the tissue slice in the culture medium.

As used herein the phrases "rotationally agitated facilitating intermittent submersion of the tissue slice in the culture medium" or "agitating in a rotation facilitating intermittent submersion of the tissue slice in the culture medium" refers to agitation which allows periodic submersion of the tissue slice in the medium such that facilitates nutrients and gas diffusion throughout the medium and through the tissue slice.

According to specific embodiments, the agitation is orbital agitation.

According to specific embodiments the agitation is an angled agitation, e.g. an angle of 30° - 45 °.

According to specific embodiments, the agitation is effected by an inclined rotator (such as the MD2500 incubation unit commercially available from Alabama Research and Development)

According to specific embodiments the agitation frequency is 50 - 200 rpm, 50 - 150 rpm or 50 - 100 rpm.

According to specific embodiment the agitation frequency is about 70 rpm.
According to some embodiments, EVOC is described in WO2018/185760 to the present inventors, which is hereby incorporated by reference in its entirety.

It is expected that during the life of a patent maturing from this application many relevant MEK inhibitors and Src inhibitors will be developed and the scope of the terms is intended to include all such new technologies *a priori.*

As used herein the term "about" refers to ± 10 %.

The terms "comprises", "comprising", "includes", "including", "having" and their conjugates mean "including but not limited to".

The term "consisting of" means "including and limited to".

The term "consisting essentially of" means that the composition, method or structure may include additional ingredients, steps and/or parts, but only if the additional ingredients, steps and/or parts do not materially alter the basic and novel characteristics of the claimed composition, method or structure.

As used herein, the singular form "a", "an" and "the" include plural references unless the context clearly dictates otherwise. For example, the term "a compound" or "at least one compound" may include a plurality of compounds, including mixtures thereof.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

As used herein, the term "treating" includes abrogating, substantially inhibiting, slowing or reversing the progression of a condition, substantially ameliorating clinical or aesthetical symptoms of a condition or substantially preventing the appearance of clinical or aesthetical symptoms of a condition.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

Various embodiments and aspects of the present invention as delineated hereinabove and as claimed in the claims section below find experimental support in the following examples.

### EXAMPLES

Reference is now made to the following examples, which together with the above descriptions illustrate some embodiments of the invention in a non limiting fashion.

### MATERIALS AND METHODS

***Cell culture and screen.*** Cell lines T84 (CCL-248), SW48 (CCL-231), LS411N (CRL-2159), SW480 (CCL-228), SW620 (CCL-227), HCT15 (CCL_225), HCT116 (CCL-247) and LS 174T (CL-188) were purchased from ATCC and were grown in DMEM supplemented with 10% serum, 1% sodium pyruvate (11 mg ml-1; Biological Industries, 03-042-1B) and penicillin/streptomycin. Stable expression of enhanced green fluorescent protein (eGFP) was achieved in all cell lines through lentiviral infection. For in vitro screens, cells were seeded at day 0 into 384-well plates (Corning, 3712) at a density of 2,000 cells per well in 50 µl of medium using an EL406 liquid dispenser (BioTek). Three replicate plates were seeded for each cell line. Throughout the screen, cells were incubated at 37 °C in a humidified incubator with 5% CO2. Single or combined drugs were arrayed into 384-well drug plates using the Echo Acoustic liquid handler (Labcyte). Each drug plate was used for all three replicate plates and contained at least six wells of DMSO as control treatment as well as single-drug treatment for all drugs. Drugs were added to the plate using a CyBio well Vario (CyBio) on day 1. On day 4, medium was replaced with fresh medium, and fresh drugs were added. GFP fluorescence was measured at days 1, 4 and 7 using Cytation3 (BioTek). PC9 NSCLC cells stably expressing eGFP as described above were counted daily by the Operetta high content imaging system (Perkin Elmer). The number of cells was normalized to day 0 by dividing the number of cells each day by the number of cells at day 0.

***Western blotting.*** Cells were grown as described above, seeded in 6-cm plates and treated with drugs as labeled for 24 h. Drug dose was determined by the *in vitro* screens described in Figure 3A; the IC20 was determined by screen 1, and the most synergistic dose (0.5 × IC20, IC20 or 2 × IC20) was determined by screen 3. The most synergistic dose was used for each drug separately and in combination. The cells were washed twice with cold PBS and then collected by scraping on ice. Cells in PBS were then centrifuged (450g, 4 °C, 5 min), and after the removal of the supernatant, 1× RIPA buffer was added. Sample buffer (2x) was added to the lysate, and the samples were resolved on a 12% SDS-PAGE gel and transferred to nitrocellulose membranes (Sigma-Aldrich). The membranes were blocked using Intercept Blocking Buffer (Li-Cor) and immunoblotted with the following antibodies diluted in Intercept Antibody Diluent (Li-Cor): pSrc (Tyr 416; 2101, Cell Signaling; 1:1,000), pERK1/ERK2 (Thr 202/Tyr 204; 4370, Cell Signaling; 1:1,000), total ERK1/ERK2 (9107, Cell Signaling; 1:1,000), total Src (2110, clone L4A1, Cell Signaling; 1:1,000), pAKT (S473; 4060, clone D9E, Cell Signaling; 1:1,000), pan total AKT (2920, clone 40D4, Cell Signaling; 1:2,000) and heat shock protein 90 (HSP90; 4874, Cell Signaling; 1:1,000). The following secondary antibodies at a concentration of 1:1,000 were then added: IRDye 800CW goat anti-mouse (926-32210) goat anti-rabbit (926-32211) or 680RD goat anti-mouse (926-68070) goat anti-rabbit (926-98071) (Li-Cor). Near-infrared fluorescence was detected with the Odyssey Infrared Imaging System (Li-Cor), and signal intensity was quantified with ImageJ software (version 1.53a).

***Determination of synergy in vitro.*** Net endpoint fluorescence was calculated by subtracting baseline GFP fluorescence (day 0) from endpoint fluorescence (day 7) for each well. For each treatment, we calculated the effect on cell viability by averaging the net endpoint fluorescence of this treatment from the three replicate plates and dividing it by the average net endpoint fluorescence of DMSO-treated wells. The Bliss index was calculated as the ratio between observed effect of combined drug treatment and expected effect (based on the calculated effect of combining single-drug treatment).

***Tissue acquisition and EVOC.*** Female NOD *scid* gamma (NSG) mice (4-6 months old) bearing PDX tumors were purchased from Jackson Laboratories. Mice arrived to the facility when the tumors had grown sufficiently to be apparent (approximately 60 mm³) and were maintained until the maximal permitted tumor volume of 1,000 mm³ was achieved. Mice were killed with CO₂, and tumor tissues were removed and placed in ice-cold PBS. Tumors were sliced into 250-µm-thick slices using a vibratome (VF300, Precisionary Instruments) and placed in six-well plates on titanium grids (MA0036, Alabama R&D) with 4 ml of DMEM/F12 medium (supplemented with 5% fetal calf serum, 100 IU ml⁻¹ penicillin with 100 µg ml-1 streptomycin, 2.5 µg ml⁻¹ amphotericin B, 50 mg ml⁻¹ gentamicin sulfate and 100 µl ml-1 l-glutamine). Tissue was cultured at 37 °C with 5% CO₂ and 80% O₂ on an orbital shaker (TOU-120N, MRC) at 70 r.p.m. The following day, tissue was treated with drugs as indicated for 96 h (medium was changed after 48 h and fresh drug was added), followed by formalin fixing and paraffin embedding after overnight fixation in 4% paraformaldehyde. Note that duplicates and triplicates were assigned randomly to mimic heterogeneity within the tumor tissue in vivo.

Human tumor tissue, either from primary colon cancer or metastatic lesions, was obtained from individuals at the time of operation or at the time of tissue core biopsy. After resection, the fresh tissue was placed in ice-cold PBS for immediate transfer to the lab for EVOC. Specimens were coded anonymously before their arrival to the lab. Forty-six individuals were enrolled in the study and had sufficient tumor volume at operation or accessible tumors for core biopsy to allow us to perform EVOC. In one individual, both primary and metastatic cancer tissue was obtained so that 47 samples in total were studied. In 14 individuals, there was either adenoma or not enough tumor tissue for evaluation. In an additional three individuals, the tissue was necrotic before slicing and EVOC. In one individual, the tissue culture was contaminated, and in one individual, the fixation was insufficient. Finally, 29 CRC primary or metastatic tumors from 28 individuals were analyzed by EVOC.

***Tissue immunohistochemistry.*** Immunohistochemistry was performed on 4-µm sections from FFPE tissue samples from EVOC. H&E staining was performed using an automated stainer. Antibody staining was performed using a Leica BondRx automated stainer. All antibodies used a citric acid decloaking step. Anti-p44/42 MAPK (ERK1/ERK2; D13.14.4E; 1:200; Cell Signaling, 4370), anti-pSrc (Tyr 416; 1:200; Cell Signaling, 2101), anti-pMEK1/MEK2 (Ser 221, 166F8; 1:200; Cell Signaling, 2338), anti-pCREB (Ser 133, 87G3; 1:200; Cell Signaling, 9198), anti-pHistone H3 (Ser 10; 1:200; Cell Signaling, 9701), anti-p53 (DO-1; 1:100; Santa Cruz, SC-126), anti-pRB (Ser 807/Ser 811; 1:400; Cell Signaling, 8516) and anti-bromodeoxyuridine (anti-BrdU; Ab-3; 1:100; Thermo Scientific, MS-1058) were used. Histological samples were scanned using the Pannoramic SCAN II (3DHistech). Caseviewer software version 2.4 (free software made available by 3DHistech) was used to take pictures of the scanned images. The intensity of pSrc and pERK in the samples was scored from 0 to 3 by a pathologist blinded to treatment results. Scores of 0 or 1 were designated as low pSrc and pERK, whereas scores of 2 or 3 were designated as high pSrc and pERK.

The following scoring system was used: score of 0, no staining; score of 1, weak and focal cytoplasmic staining; score of 2, weak and diffuse or strong and focal cytoplasmic staining; score of 3, strong and diffuse cytoplasmic staining.

***Scoring of tissue response and synergy ex vivo.*** In vivo experiments were scored by the percent reduction in fold change of tumor growth between the treated and untreated control mice as follows: NR, no response (<30% reduction); PR, partial
response (≥30% to <100% reduction); CR, complete or near-complete response (≥100% reduction).

Histologic sections were assessed for viability by an independent blinded pathologist. Tissue viability was scored on a scale from 0 to 4, where 0 is less than 10% viable tissue and 4 is more than 90% viable tissue. Scores of 3, 2 and 1 correspond to tissue viability of 61-90%, 31-60% and 10-30%, respectively. To score a combination of drugs as effective, it had to fulfill the following three criteria: (1) the viability score of the combination of drugs given together with FOLFOX or FOLFIRI was ≤1, (2) the viability score of the drug combination with chemotherapy was lower than the viability score of the chemotherapy alone, and (3) none of the drugs in the combination resulted in a viability score of 0 when applied alone.

Slices where the amount of cancer tissue was less than 10% of the entire tissue were considered to be insufficient for the evaluation of the response to drug.

***Determination of drug treatment concentrations.*** To determine the concentration of each drug to be used in the ex vivo screen, we first calibrated the effect of multiple concentrations of each drug on PDX CRC tumors. For the screens, we selected the doses that were minimally effective in killing tissue after 4 d of treatment as evaluated by H&E staining. It was found that selected drug concentrations were largely within the range of drug concentrations determined clinically in participant sera (Tables 1 and 2). In addition, when testing targeted therapies, the present inventors validated that the chosen drug concentrations were effective in inhibiting their targets. This was evaluated by immunohistochemical staining after 24 h of treatment (Figures 11A-C). Here again, the lowest drug concentration that was enough to inhibit the drug target was used.

**Table 1**

| | | *Drug Concentrations used in EVOC for fresh & PDX human tumors* | |
|---|---|---|---|
| Drug | MW | Range eliciting differential response (uM) * | In combination exps (uM) |
| Selumetinib | 457.7 | 0.25-4 | 0.5 |
| Bosutinib | 530.4 | 1-16 | 2 |
| 5-FU | 130.1 | 2-320 | 20 |
| Irinotecan | 586.7 | 30-120 | 30 |
| Oxaliplatin | 397.3 | 2-64 | 20 |
| Afatinib | 485.9 | 16-64 | 25 |
| VX-680 | 464.6 | 1-16 | 1 |
| GDC-0941 | 513.6 | 1-16 | 5 |
| H89 2HCl | 519.3 | 1-16 | 1 |
| Nutlin | 581.5 | 4-16 | 4 |
| Cisplatin | 300.0 | 0.3-30 | N/A |
| Docetaxel | 807.9 | 0.2-80 | N/A |
| Trametinib | 615.4 | 0.1-1 | N/A |
| 4-OH-Cyclophosphamide | 293.1 | 2-20 | N/A |

| | | | |
|---|---|---|---|
| *The concentrations of drug that demonstrate a difference in amount of cell death in individual patient tissues, i.e. drug concentrations are not so high that all the tissue is killed in all the patients, nor so low that none of the tissue is killed in any of the patients. Rather the tissues that are more sensitive to the drug will be killed at low concentration while tissues that are not sensitive will not be killed even at high concentration of drug. | | | |

**Table 2**

| Clinical data of max plasma concentrations in response to specific dose | | |
|---|---|---|
| **Dose** | **Plasma Concentration reported** | **Plasma Concentration (uM)** |
| 75mg | 1520ng/mL | 3.3 |
| 120mg IV | 360 ng/mL | 0.7 |
| 2500 mg/m² | 2500-3000 ug/L | 19.2-23 |
| 100-350 mg/m² | 4.043-46.164 | 6.8-78 |
| 130mg/m² | 1.5-4.8 ug/mL | 3.8-12.1 |
| 40mg | 130 ng/mL | 0.27 |
| 8-96mg/m² | 0.1-5 mM | 0.1-5 |
| 15-450mg | 0.1-2.65 mM | 0.1-2.65 |
| 200mg/kg | 50 mM | 50 |
| 100mg/m² | 11 ug/ml | 30.7 |
| 100mg/m² | 2616-6949 nM | 2.6-6.9 |
| 2.5mg | 63.2 ng/mL | 0.1 |
| 500mg/m² | 4.35 mg/mL | 15 |

***Sequencing.*** Next-generation sequencing for the detection of specific somatic DNA mutations was performed using the Oncomine Solid Tumor DNA kit (Thermo Fisher Scientific) according to the manufacturer's instructions. All tissue samples were collected from FFPE blocks after representative slides had been examined by a qualified pathologist. The next-generation sequencing clinical-grade test was performed at pathology laboratories in Israeli hospitals. The quality and amount of DNA in each sample was checked using an Ion Sphere Quality Control kit and measured using a Qubit 3.0 Fluorometer (both from Thermo Fisher Scientific). The sequencing results were analyzed using an Ion Reporter Server System (Thermo Fisher Scientific).

***Statistics and reproducibility.*** The response of PDX tissues to treatment as determined by EVOC was scored as mentioned above (see Scoring of tissue response and synergy ex vivo). Once determined, the viability scores were used to compare the difference in response between PDX models (Figures 2A-G and Figures 9A-J). A two-sided rank-sum test (Mann-Whitney) was used to determine *P* values for all the PDX models. To determine the *P* values of contingency tables (for example, responders versus non-responders for the presence of RAS mutations), a chi-squared test was used (Figures 14A-C). To determine the *P* values for the differences in pSrc staining in responders versus non-responders, a two-tailed Fisher exact test was used. To determine the *P* values for changes in pERK scores after treatment with bosutinib, a Mann-Whitney test was used. To determine *P* values for paired samples in Figures 4A-B and Figure 6A (changes in pERK), a Wilcoxon matched pairs signed-rank test was used (two sided). The intensity of the pSrc/HSP90 ratio in western blotting was compared between the two groups of CRC cell lines (four in each group) using a two-sided Student's *t*-test. No statistical method was used to predetermine sample size. In the EVOC experiments, tissues were treated with the maximum number of drug combinations possible, depending on the availability of tissue. The investigators had no knowledge of the participants' clinical course at the time of the experiment and data analysis. The available clinical data were collected by collaborators not directly involved in the experiments and blinded to the experimental results.

No data points were excluded in this study.

### EXAMPLE 1

### Optimizing ex vivo culture for prediction of in vivo drug response

Cancer drug efficacy is most often determined both *in vitro* and *ex vivo* by treating cell cultures and tissues for 24-72 hours (17). Depending on the drug, however, some cancer cells die much later. Indeed, early measurements often reflect the effect of drugs on proliferation rather than on cell killing (18). For example, by measuring daily the number of cells of the non-small cell lung cancer (NSCLC) cell line PC9 in response to both targeted and cytotoxic therapies, it was found that more than 2-3 days of treatment are necessary to separate cytotoxic from cytostatic response, and to accurately characterize the effect of treatment on cancer cell killing (Figure 1A).

Therefore the *ex vivo* tissue culture method was modified to maintain tissue viability for at least 6 days by testing various culture conditions. It was found that viability in DMEM/F12 media was superior to viability in other media types (15), and that culturing the tissue on a titanium grid with orbital shaking, which maintains the tissue at the air-liquid interface, improved tissue viability, as compared to submerging the tissue in media (Figure 1B) (19). Lastly, it was found that culturing tissue in O₂ partial pressure of 80% was the most important determinant of tissue viability (Figure 1B, Figures 8A-D). Note that capillaries in human tissues are approximately 40-80 um apart, and the required distance that oxygen must diffuse to reach cells is thus usually not more than 20-40 um (20). By contrast, the present tissue slices are 250 um thick, and therefore there is a marked drop in the oxygen partial pressure between the surface and the center of the tissue slices. Starting with a relatively high oxygen partial pressure in the culturing chamber thus enables better representation of the physiological oxygen partial pressure in tumor slices.

Overall, using these modified conditions, 147 human tumors were analyzed originating from multiple tumor types, both from resections and from core biopsy samples (Figures 1C,D, not shown). Thirty-three out of the 147 samples (22.4%) had no cancer cells in the tissue. Of the first half of the samples, 27.4% (20/73) were without cancer cells, whereas only 17.5% of the samples recruited in the second half of the study (13/74) were found not to have cancer cells. Six samples (4.1%) were found to be necrotic on arrival. After removing samples for which no cancer tissue was present for evaluation, and tissues that were necrotic on arrival to the lab, the present inventors were able to evaluate 108 samples. 104 of these samples were successfully cultured for five days (96.3%).

CRC samples were tested for sensitivity to commonly used anti-cancer treatments. It was found that the viability of all eight tumors was maintained over five days of culture, that each tumor responded differently to the different drugs, and that different tumors responded very differently to the same drug (Figures 1E,F). These results recapitulate the known variable clinical response expected from colorectal cancer patients (21).

To test that the *ex vivo* culture can accurately predict the response of tumors to the same treatment *in vivo,* seven patient-derived xenograft (PDX) tumor models originating from colon, lung, and breast cancer, with an observed wide range of *in vivo* responses to various anti-cancer drugs were selected. *In vivo* and *ex vivo* response of 16 pairs of PDX models and their clinically relevant drugs were compared. Each model was tested against a range of drug concentrations *ex vivo,* with the highest concentration chosen to be at least as high as the highest clinically relevant drug concentration (not shown), and still showing differential drug response between the different models. It was possible to demonstrate that in 15 out of the 16 models tested, EVOC could correctly differentiate among no response, partial response, and complete or near-complete response, as observed *in vivo* (Figures 2A-G, Figures 9A-J and not shown). Overall, it was established that the EVOC system can be used to culture multiple tumor types for at least 5 days, and that it is highly accurate in predicting *in vivo* results of PDX models.

### EXAMPLE 2

### In vitro screens to prioritize new synergistic drug combinations

While EVOC technology can potentially be a much more accurate biomarker of the response of human tumors to drugs than *in vitro* models, the throughput of *ex vivo* culturing is limited compared to that of *in vitro* drug screens. Therefore, the present inventors sought to identify high-throughput *in vitro* drug screens so as to prioritize the most synergistic drug combinations to be tested *ex vivo.*

The effect of all 276 possible dual-drug combinations of 24 chosen drugs in eight colorectal cancer cell lines were tested (Figures 3A,B). The IC20 concentration was determined for each of the 24 drugs in each of the eight cell lines (not shown). All cell lines were then treated with the 276 dual-drug combinations at their IC20, and 21 combinations that proved to be synergistic were prioritized for a validation screen (Figures 3A,C, Figure 10 and not shown). Then, the effect of all 21 combinations was tested at IC20, twice IC20, and half IC20 for each drug, so that each combination was tested 9 times in every cell line (Figure 3A,C and Figure 10). Synergy was determined by a Bliss score of <0.6, and the top seven candidates were chosen for further evaluation, as they were synergistic in at least 3 cell lines, apart from one combination that was highly synergistic in only two cell lines (Figure 3D). Based on the historically low success rate in validating *in vitro* results in clinical trials, it was expected none or only one of these combinations would show efficacy in the present *ex vivo* preclinical study.

### EXAMPLE 3

### MEK/Src inhibitory combination shows ex vivo effective in human CRC

Two of the seven prioritized combinations (Figure 3D), irinotecan and olaparib, and oxaliplatin and palbociclib, although effective in preclinical mouse models (22, 23), failed clinical trials (NCT01522989, NCT03709680) (24), and were discarded from further evaluation. Of the remaining five combinations, three were previously shown to have synergistic effect *in vitro* and in preclinical models of CRC cell lines (25, 26, 27). As none of these five combinations were ever tested on primary human CRC, all five were evaluated *ex vivo.* The drug concentrations used in EVOC were determined by testing a dose curve of each drug separately on a CRC PDX tumor model. The minimal dose needed to kill ~10-20% of the cancer cells after four days of treatment was determined, and in the case of targeted therapies, the minimal dose that affected the primary drug target after 24 hours of treatment (Figures 11A-C). It was found that the chosen drug concentrations were predominantly within the reported range of the published human plasma concentrations (not shown).

29 colorectal adenocarcinomas from 28 patients were examined by EVOC. Sixteen samples were taken from the primary tumor, nine from liver metastases, three from peritoneal metastases, and one from lung metastasis. From patient 43, samples were from both the primary tumor and from a liver metastasis. For each tumor sample, the effect of up to three drug combinations was tested. Because it was assumed that these potential combinations will be given in addition to the widely used cytotoxic drug pairs 5-FU/oxaliplatin (FOLFOX) or 5-FU/irinotecan (FOLFIRI), all five combinations were tested with and without these cytotoxic drug pairs (Figures 12A-E).

Drug combinations were considered to be effective if they met all of the following three criteria: (1) None of the drugs alone killed all cancer cells (viability score 0), demonstrating that the sensitivity of the tissue was to the drug combination rather than to only one of the drugs. (2) Adding the drug combination to either FOLFOX or FOLFIRI resulted in a lower viability score than the effect of FOLFOX or FOLFIRI alone. (3) Tissues treated with the drug combination and with either FOLFOX or FOLFIRI resulted in a substantial pathological response (viability score 0 or 1).

Overall it was found that 50% (13/26) of the tumors responded to the combination of selumetinib and bosutinib, inhibitors of MEK and Src respectively (Figures 4A-D, Figures 13A-B). The combination of the MEK inhibitor selumetinib and the Src inhibitor bosutinib, was even more effective when combined with FOLFOX or FOLFIRI (Figure 4B). Using the Src inhibitor dasatinib instead of bosutinib or the MEK inhibitor trametinib instead of selumetinib, resulted in similar sensitivity (Figures 14A-C). The other four combinations, including the combination of bosutinib and a PI3K inhibitor, showed either no response or a response in only a small minority of the tumors (Figure 4A, Figures 12A-E and Figures 15A-C), and were not investigated further. Of note, clinical data on the response to FOLFOX or FOLFIRI was available for only four of the patients, and in all of these cases this matched the EVOC results (Figure 16).

### EXAMPLE 4

### High pSrc is a biomarker for sensitivity to MEK/Src inhibition

Next, the present inventors wanted to identify the molecular mechanism that differentiates between CRC tumors that are sensitive to dual MEK/Src inhibition from those that are not. It was previously suggested by Anderson et al. (25) that the presence of concomitant *KRAS* and *PIK3CA* mutations is a biomarker for sensitivity to MEK/Src inhibition in CRC cell lines. Unlike the reported finding in cell lines, this correlation was not observed in the present EVOC primary human tumors. Of the 24 patients with known mutation status, 15 patients (62.5%) had a *KRAS* mutation and seven (29.2%) also had a *PIK3CA* mutation, with double mutants present almost equally between responders (4/12) and non-responders (3/12) (not shown) (p-value=1). Instead, the present inventors found enrichment of activating *RAS* mutations (*KRAS* or *NRAS*) in non-responders to MEK/Src inhibition (11/12 of non-responders vs. 6/12 of responders, p=0.025 by Chi-square test).

As neither the *RAS* nor the *RAS*/*PIK3CA* mutational status fully explained the difference between responders and non-responders, other molecular mechanisms were sought that may account for this difference. It was previously reported that the activating phosphorylation of Src (pSrc) at tyrosine 419 is present in 38-84% of CRC tumors, and that the presence of pSrc increases with tumor progression (28, 29). Activation of Src can lead to the activation of multiple downstream signaling, including MAPK pathway, PI3K-AKT, and the Hippo pathway, as well as to the activation of EGFR, supporting tumor progression and survival (Figure 5) (30). It was hypothesized that the subgroup of CRC patients who respond to MEK/Src inhibition are the same patients who have high pSrc in their tumors. In agreement with this model, a striking positive correlation was found between pSrc in non-treated CRC tumors and their response to MEK/Src inhibition. Whereas 81.8% (9/11) of the MEK/Src-sensitive tumors had a high pSrc score (2 or 3) by immunohistochemistry staining, only 10% of the resistant tumors (1/10) had a high pSrc score (p=0.002) (Figures 6A,B). This finding was not recapitulated in the CRC cell lines tested in vitro (Figures 17A-B; *P* = 0.21 by two-tailed Student's *t*-test), emphasizing again the strength of using EVOC as a preclinical model.

### EXAMPLE 5

### MAPK signaling in tumors with high pSrc is dependent on Src activity

Based on these results it was hypothesized that in patients with high pSrc, most of the MAPK flux is driven by Src signaling, while in patients with low pSrc, the MAPK signaling is not driven by Src activity (Figure 5). To validate this hypothesis, the *ex vivo* effect of Src inhibition as a single agent was tested in 16 CRC tumors for which enough tissue was available to evaluate pERK. It was found that while treatment with the Src inhibitor bosutinib resulted in a low pERK score (0 or 1) in 7 out of 8 responders, pERK score remained high (2 or 3) in 6 of 8 non-responder tumors that were tested (p=0.003) (Figures 6A-B).

To further demonstrate the importance of Src activity in driving the flux in the MAPK pathway of responders to MEK/Src inhibition, the present inventors used MEK inhibition to block the flux in the MAPK pathway and reduce many of the negative feedbacks associated with MAPK pathway activity. It has been shown that pMEK is upregulated as a result of MEK inhibition, reflecting a disinhibition of upstream signaling (31). As expected, the present inventors observed upregulation of pMEK in response to MEK inhibition in both responders and non-responders, reflecting the higher flux through RAF (Figures 7A-B) (32). Importantly, the addition of SRC inhibition was able to repress selumetinib-induced increase in pMEK only in responders, further supporting the proposition that in responders, the MAPK pathway is driven mostly by Src activity. By contrast, Src inhibition did not affect pMEK under MEK inhibition in non-responders, reflecting the lack of contribution of Src to the flux in the MAPK pathway of these tumors (Figures 7A-B).

The present *ex vivo* results demonstrate that dual MEK/Src inhibition in responders is superior to Src inhibition alone (Figures 4A-D), suggesting that a minor MAPK flux is still present in responders under Src inhibition. Accordingly, it was found that adding MEKi to Srci in responders eliminated the small pERK signal that is still present under single therapy with Src inhibition (Figures 6A-B).

### KRASG12 mutation predicts resistance to MEK/Src inhibition.

Lastly, the present inventors sought to understand how patients with a *KRAS* mutation can still respond to MEK/Src inhibition (6 of 12 responders had *KRAS* mutation), as it would be expected that the mutated *KRAS* can signal even in the absence of Src signaling. Recent works have demonstrated that different *KRAS* mutations may have different signaling effects. *KRAS* mutations in codon 12 not only induce KRAS activation by inhibiting its GTPase activity, but they also inhibit NF1 activity, resulting in activation of the wild-type KRAS, NRAS, and HRAS proteins (33, 34). It is therefore expected that in tumors with *KRAS* codon 12 mutations, inhibition of Src would have only a minimal effect. By contrast, it was demonstrated that mutations in codon 13 of *KRAS* (e.g., G13D) or other non-codon 12 mutations, have no effect on NF1 and thus on the other wild-type RAS proteins. It was further demonstrated that colorectal tumors with *KRAS* codon 13 mutations, unlike tumors with codon 12 mutations, are sensitive to EGFR inhibition, reflecting the need of *KRAS* codon 13 mutated cancers for additional oncogenic signaling originating from the EGFR (35). It was therefore hypothesized that responder patients with a *KRAS* mutation would be largely depleted of *KRAS* codon 12 mutations. Examining the *KRAS* mutations in the patients, it was found that while 9 out of the 10 (90%) non-responder tissues with a *KRAS* mutation had a codon 12 mutation, only one out of six responder tissues (16.6%) had a *KRAS* codon 12 mutation (p=0.0076). Therefore, it is concluded that inhibition of MEK/Src in patients with high pSrc is expected to be effective in the presence of non-codon 12 *KRAS* mutations, but will largely not be effective in patients who have a *KRAS* codon 12 mutation (Figure 5).

Overall, the data suggest that approximately 50 % of CRC patients may be sensitive to MEK/Src inhibition and that multiple biomarkers may help to pre-identify this subset of patients. While EVOC may not be currently available in the clinical setting, pSRC levels and KRAS status may be sufficient to select the majority of patients in this subset. Indeed, in the present study, all patients (n=8) with positive pSRC and lack of KRAS G12 mutation responded to MEK/Src inhibition ex vivo while all patients with lack of pSRC but with KRAS G12 mutation (n=5) did not respond to the combination (not shown). For a third of the patients that are expected to have a conflicting pSRC and KRAS status (6 of 19, data not shown), EVOC may be used as a deciding biomarker. EVOC can also give additional functional support for the expected response of patients with high pSRC and lack of KRAS G12 mutation to MEK/Src inhibition and thus be used to even further enrich clinical trials for expected responders.

Although the invention has been described in conjunction with specific embodiments thereof, it is evident that many alternatives, modifications and variations will be apparent to those skilled in the art. Accordingly, it is intended to embrace all such alternatives, modifications and variations that fall within the scope of the appended claims.

### REFERENCES

### (other references are cited in the document)

1. Quail DF, Joyce JA. Microenvironmental regulation of tumor progression and metastasis. Nat Med. 19, 1423-37 (2013).
2. Jaiswal R, Sedger LM. Intercellular Vesicular Transfer by Exosomes, Microparticles and Oncosomes - Implications for Cancer Biology and Treatments. Front Oncol. 9, 125 (2019).
3. Goulet CR, Champagne A, Bernard G, Vandal D, Chabaud S, Pouliot F, et al. Cancer-associated fibroblasts induce epithelial-mesenchymal transition of bladder cancer cells through paracrine IL-6 signalling. BMC Cancer 19, 137 (2019).
4. Geller LT, Barzily-Rokni M, Danino T, Jonas OH, Shental N, Nejman D, et al. Potential role of intratumor bacteria in mediating tumor resistance to the chemotherapeutic drug gemcitabine. Science 357, 1156-60 (2017).
5. Garattini S, Grignaschi G. Animal testing is still the best way to find new treatments for patients. Eur J Intern Med. 39, 32-5 (2017).
6. Hutchinson L, Kirk R. High drug attrition rates-where are we going wrong? Nat Rev Clin Oncol. 8, 189-90 (2011).
7. Wong CH, Siah KW, Lo AW. Estimation of clinical trial success rates and related parameters. Biostatistics 20, 273-86 (2019).
8. Van Cutsem E, Cervantes A, Nordlinger B, Arnold D, on behalf of the ESMO Guidelines Working Group. Metastatic colorectal cancer: ESMO Clinical Practice Guidelines for diagnosis, treatment and follow-up. Ann Oncol. 25, iiil-9 (2014).
9. Vogel A, Hofheinz RD, Kubicka S, Arnold D. Treatment decisions in metastatic colorectal cancer - Beyond first and second line combination therapies. Cancer Treat Rev. 59, 54-60 (2017).
10. Guinney J, Dienstmann R, Wang X, de Reyniès A, Schlicker A, Soneson C, et al. The consensus molecular subtypes of colorectal cancer. Nat Med. 21, 1350-6 (2015).
11. Dienstmann R, Vermeulen L, Guinney J, Kopetz S, Tejpar S, Tabernero J. Consensus molecular subtypes and the evolution of precision medicine in colorectal cancer. Nat Rev Cancer 17,79-92 (2017).
12. Wang W, Kandimalla R, Huang H, Zhu L, Li Y, Gao F, et al. Molecular subtyping of colorectal cancer: Recent progress, new challenges and emerging opportunities. Semin Cancer Biol. 55, 37-52 (2019).
13. Hickman JA, Graeser R, de Hoogt R, Vidic S, Brito C, Gutekunst M, et al. Three-dimensional models of cancer for pharmacology and cancer cell biology: Capturing tumor complexity in vitro/ex vivo. Biotechnol J. 9, 1115-28 (2014).
14. Majumder B, Baraneedharan U, Thiyagarajan S, Radhakrishnan P, Narasimhan H, Dhandapani M, et al. Predicting clinical response to anticancer drugs using an ex vivo platform that captures tumour heterogeneity. Nat Commun. 6, 6169 (2015).
15. Grosso SHG, Katayama MLH, Roela RA, Nonogaki S, Soares FA, Brentani H, et al. Breast cancer tissue slices as a model for evaluation of response to rapamycin. Cell Tissue Res. 352, 671-84 (2013).
16. Kern MA, Haugg AM, Eiteneuer E, Konze E, Drebber U, Dienes HP, et al. Ex vivo analysis of antineoplastic agents in precision-cut tissue slices of human origin: effects of cyclooxygenase-2 inhibition in hepatocellular carcinoma. Liver Int. 26, 604-12 (2006).
17. Larsson P, Engqvist H, Biermann J, Werner Rönnerman E, Forssell-Aronsson E, Kovács A, et al. Optimization of cell viability assays to improve replicability and reproducibility of cancer drug sensitivity screens. Sci Rep. 10, 5798 (2020).
18. Fallahi-Sichani M, Honarnejad S, Heiser LM, Gray JW, Sorger PK. Metrics other than potency reveal systematic variation in responses to cancer drugs. Nat Chem Biol. 9, 708-14 (2013).
19. Vaira V, Fedele G, Pyne S, Fasoli E, Zadra G, Bailey D, et al. Preclinical model of organotypic culture for pharmacodynamic profiling of human tumors. Proc Natl Acad Sci. 107, 8352-6 (2010).
20. Place TL, Domann FE, Case AJ. Limitations of oxygen delivery to cells in culture: An underappreciated problem in basic and translational research. Free Radic Biol Med. 113, 311-22 (2017).
21. Pasetto LM, Jirillo A, Iadicicco G, Rossi E, Paris MK, Monfardini S. FOLFOX Versus FOLFIRI: A Comparison of Regimens in the Treatment of Colorectal Cancer Metastases. Anticancer Res. 25(1B), 563-76 (2015).
22. Genther Williams SM, Kuznicki AM, Andrade P, Dolinski BM, Elbi C, O'Hagan RC, et al. Treatment with the PARP inhibitor, niraparib, sensitizes colorectal cancer cell lines to irinotecan regardless of MSI/MSS status. Cancer Cell Int. 15, 14 (2015).
23. Tentori L, Leonetti C, Scarsella M, Muzi A, Mazzon E, Vergati M, et al. Inhibition of poly(ADP-ribose) polymerase prevents irinotecan-induced intestinal damage and enhances irinotecan/temozolomide efficacy against colon carcinoma. FASEB J. 20, 1709-11 (2006).
24. Chen EX, Jonker DJ, Siu LL, McKeever K, Keller D, Wells J, et al. A Phase I study of olaparib and irinotecan in patients with colorectal cancer: Canadian Cancer Trials Group IND 187. Invest New Drugs 34, 450-7 (2016).
25. Anderson GR, Winter PS, Lin KH, Nussbaum DP, Cakir M, Stein EM, et al. A Landscape of Therapeutic Cooperativity in KRAS Mutant Cancers Reveals Principles for Controlling Tumor Evolution. Cell Rep. 20, 999-1015 (2017).
26. Astsaturov I, Ratushny V, Sukhanova A, Einarson MB, Bagnyukova T, Zhou Y, et al. Synthetic Lethal Screen of an EGFR-Centered Network to Improve Targeted Therapies. Sci Signal 3, ra67-ra67 (2010).
27. Wang H, Wang S, Nan L, Yu D, Agrawal S, Zhang R. Antisense anti-MDM2 mixed-backbone oligonucleotides enhance therapeutic efficacy of topoisomerase I inhibitor irinotecan in nude mice bearing human cancer xenografts: In vivo activity and mechanisms. Int J Oncol. 20, 745-52 (2002).
28. Martínez-Pérez J, Lopez-Calderero I, Saez C, Benavent M, Limon ML, Gonzalez-Exposito R, et al. Prognostic relevance of Src activation in stage II-III colon cancer. Hum Pathol. 67, 119-25 (2017).
29. Talamonti MS, Roh MS, Curley SA, Gallickt GE. Increase in Activity and Level of pp6Oc-erc in Progressive Stages of Human Colorectal Cancer. J Clin Invest. 1, 53-60 (1993).
30. Si Y, Ji X, Cao X, Dai X, Xu L, Zhao H, et al. Src Inhibits the Hippo Tumor Suppressor Pathway through Tyrosine Phosphorylation of Lats1. Cancer Res. 77, 4868-80 (2017).
31. Yeh TC, Marsh V, Bernat BA, Ballard J, Colwell H, Evans RJ, et al. Biological Characterization of ARRY-142886 (AZD6244), a Potent, Highly Selective Mitogen-Activated Protein Kinase Kinase 1/2 Inhibitor. Clin Cancer Res. 13, 1576-83 (2007).
32. Wu P-K, Park J-I. MEK1/2 Inhibitors: Molecular Activity and Resistance Mechanisms. Semin Oncol. 42, 849-62 (2015).
33. McFall T, Diedrich JK, Mengistu M, Littlechild SL, Paskvan KV, Sisk-Hackworth L, et al. A systems mechanism for KRAS mutant allele-specific responses to targeted therapy. Sci Signal 12, eaaw8288 (2019).
34. McFall T, Stites EC. A mechanism for the response of KRAS G13D expressing colorectal cancers to EGFR inhibitors. Mol Cell Oncol. 7, 1701914 (2020).
35. Mao C, Huang Y-F, Yang Z-Y, Zheng D-Y, Chen J-Z, Tang J-L. KRAS p.G13D mutation and codon 12 mutations are not created equal in predicting clinical outcomes of cetuximab in metastatic colorectal cancer. Cancer 119, 714-21 (2013).
36. Sönnichsen R, Hennig L, Blaschke V, Winter K, Körfer J, Hähnel S, et al. Individual Susceptibility Analysis Using Patient-derived Slice Cultures of Colorectal Carcinoma. Clin Colorectal Cancer 17, e189-99 (2018).
37. Donnadieu J, Lachaier E, Peria M, Saidak Z, Dakpe S, Ikoli J-F, et al. Short-term culture of tumour slices reveals the heterogeneous sensitivity of human head and neck squamous cell carcinoma to targeted therapies. BMC Cancer [Internet]. 2016 [cited 2017 Jun 8];16. Available from: www(dot)bmccancer(dot)biomedcentral(dot)com/articles/10(com)1186/s12885-016-2318-x
38. Begley CG, Ellis LM. Raise standards for preclinical cancer research. Nature 483, 531-3 (2012).
39. Hanahan D, Weinberg RA. Hallmarks of Cancer: The Next Generation. Cell 144, 646-74 (2011).
40. Ben-David U, Ha G, Tseng Y-Y, Greenwald NF, Oh C, Shih J, et al. Patient-derived xenografts undergo mouse-specific tumor evolution. Nat Genet. 49, 1567-75 (2017).
41. Lee MW, Miljanic M, Triplett T, Ramirez C, Aung KL, Eckhardt SG, et al. Current methods in translational cancer research. Cancer Metastasis Rev [Internet]. 2020 [cited 2020 Sep 17]; Available from: www(dot)link(dot)springer(dot)com/10(dot)1007/s10555-020-09931-5
42. Lu Y, Dong B, Xu F, Xu Y, Pan J, Song J, et al. CXCL1-LCN2 paracrine axis promotes progression of prostate cancer via the Src activation and epithelial-mesenchymal transition. Cell Commun Signal. 17, 118 (2019).
43. Shields DJ, Murphy EA, Desgrosellier JS, Mielgo A, Lau SKM, Barnes LA, et al. Oncogenic Ras/Src cooperativity in pancreatic neoplasia. Oncogene 30, 2123-34 (2011).
44. Morton JP, Karim SA, Graham K, Timpson P, Jamieson N, Athineos D, et al. Dasatinib Inhibits the Development of Metastases in a Mouse Model of Pancreatic Ductal Adenocarcinoma. Gastroenterology 139, 292-303 (2010).
45. Trevino JG, Summy JM, Lesslie DP, Parikh NU, Hong DS, Lee FY, et al. Inhibition of Src Expression and Activity Inhibits Tumor Progression and Metastasis of Human Pancreatic Adenocarcinoma Cells in an Orthotopic Nude Mouse Model. Am J Pathol. 168, 962-72 (2006).
46. Liu P, Wang Y, Li X. Targeting the untargetable KRAS in cancer therapy. Acta Pharm Sin B. 9, 871-9 (2019).
47. Irby RB, Yeatman TJ. Role of Src expression and activation in human cancer. Oncogene 19, 5636-42 (2000).
48. Lake D, Corrêa SAL, Müller J. Negative feedback regulation of the ERK1/2 MAPK pathway. Cell Mol Life Sci. 73, 4397-413 (2016).
49. Chen J, Elfiky A, Han M, Chen C, Saif MW. The Role of Src in Colon Cancer and Its Therapeutic Implications. Clin Colorectal Cancer 13, 5-13 (2014).
50. Montero JC, Seoane S, Ocana A, Pandiella A. Inhibition of Src Family Kinases and Receptor Tyrosine Kinases by Dasatinib: Possible Combinations in Solid Tumors. Clin Cancer Res. 17, 5546-52 (2011).
51. Parseghian CM, Parikh NU, Wu JY, Jiang Z-Q, Henderson L, Tian F, et al. Dual Inhibition of EGFR and c-Src by Cetuximab and Dasatinib Combined with FOLFOX Chemotherapy in Patients with Metastatic Colorectal Cancer. Clin Cancer Res. 23, 4146-54 (2017).

## Claims

1. A combination comprising a therapeutically effective amount of a Mitogen/extracellular signal-regulated kinase (MEK) inhibitor and a SRC Proto-Oncogene, Non-Receptor Tyrosine Kinase (SRC) inhibitor for use in treating colorectal cancer (CRC), wherein cells of the CRC express an amount of SRC p419 above a predetermined level and do not express a KRAS G12 mutation.

2. An *ex-vivo* method of selecting treatment to colorectal cancer (CRC) for a subject in need thereof, the method comprising determining a level of SRC Proto-Oncogene, Non-Receptor Tyrosine Kinase (SRC) p419 and a RAS mutation to exclude a KRAS G12 mutation in cancer cells of the subject, wherein a SRC p419 level above a predetermined level and an absence of a KRAS G12 mutation, is indicative of an efficacious treatment with of a Mitogen/extracellular signal-regulated kinase (MEK) inhibitor and a SRC inhibitor.

3. The method of claim 2, further comprising testing sensitivity to said treatment in an *ex-vivo* organ culture (EVOC) model.

4. The method of claim 3, comprising any of:
(i) testing sensitivity to said MEK inhibitor and said SRC inhibitor in said EVOK model by monitoring cell killing;
(ii) testing sensitivity to said MEK inhibitor and said SRC inhibitor in cells of said EVOK model by determining a level of MEK phosphorylation (pMEK), wherein a level below a predetermined or control is indicative of an efficacious treatment; and/or
(iii) testing sensitivity to said SRC inhibitor in cells of said EVOK model by determining level ERK1/2 phosphorylation (pERK1/2), wherein a level below a predetermined or control is indicative of an efficacious treatment.

5. The method of any one of claims 3 and 4, wherein said EVOC model comprises culturing in a culture medium a precision-cut tissue slice of said CRC placed on a tissue culture insert, wherein said precision-cut tissue slice is maintained in a highly oxygenated atmosphere containing about 80 % oxygen and wherein said culture is rotationally agitated facilitating intermittent submersion of said tissue slice in said culture medium.

6. The method of claim 5, wherein said culture is in a tissue culture plate.

7. The method of any one of claims 5 or 6, wherein said culture medium comprises DMEM/F12.

8. The method of any one of claims 5-7, wherein said culturing is effected for at least 4 or 5 days.

9. The method of any of claims 5-8, wherein said slice is in direct contact with said tissue culture insert.

10. The method of any one of claims 5-9, wherein said tissue culture insert is a titanium grid insert.

11. The combination for use of claim 1, wherein said cancer cells exhibit at least one of:
(i) sensitivity to said MEK inhibitor and said SRC inhibitor in an EVOK model as monitored by cell killing;
(ii) sensitivity to said MEK inhibitor and said SRC inhibitor in an EVOK model based on the level of MEK phosphorylation (pMEK), wherein a level below a predetermined or control is indicative of an efficacious treatment; and/or
(iii) sensitivity to said SRC inhibitor in an EVOK model based on the level of ERK1/2 phosphorylation (pERK1/2), wherein a level below a predetermined or control is indicative of an efficacious treatment.

12. The method or combination for use of any one of claims 1-10, wherein said MEK inhibitor is selected from the group consisting of trametinib and selumetinib.

13. The method or combination for use of any one of claims 1-12, wherein said SRC inhibitor is selected from the group consisting of bosutinib, dasatinib, saracotinib, ponatinib, TPX-0022 and 1-NM-PP1.

14. The method or combination for use of any one of claims 1-13, further comprising treatment with Folfox or Folfiri.

15. The method or combination for use of any one of claims 1-14, wherein said CRC is selected from the group consisting of locally advanced, metastatic CRC and a recurrent disease.

## Patentansprüche

1. Kombination, umfassend eine therapeutisch wirksame Menge eines Mitogens/extrazellulären signalregulierten Kinase (MEK)-Inhibitors und eines SRC-Protoonkogen, Nicht-Rezeptor-Tyrosinkinase (SRC)-Inhibitors zur Verwendung bei der Behandlung von Darmkrebs (CRC), wobei die Zellen des CRC eine Menge an SRC p419 über einem vorbestimmten Niveau exprimieren und keine KRAS G12-Mutation exprimieren.

2. Ex vivo-Verfahren zur Auswählen einer Behandlung für Darmkrebs (CRC) für einen Patienten, der dessen bedarf, wobei das Verfahren die Bestimmung eines Niveaus von SRC-Protoonkogen, Nicht-Rezeptor-Tyrosinkinase (SRC) p419 und einer RAS-Mutation umfasst, um eine KRAS G12-Mutation in Krebszellen des Patienten auszuschließen, wobei ein SRC p419-Niveau über einem vorbestimmten Niveau und das Fehlen einer KRAS G12-Mutation auf eine wirksame Behandlung mit einem Mitogen/extrazellulären signalregulierten Kinase (MEK)-Inhibitor und einem SRC-Inhibitor hinweisen.

3. Verfahren nach Anspruch 2, ferner umfassend ein Testen der Empfindlichkeit gegenüber der Behandlung in einem ex vivo-Organkulturmodell (EVOC).

4. Verfahren nach Anspruch 3, umfassend Folgende:
(i) Testen der Empfindlichkeit gegenüber dem MEK-Inhibitor und dem SRC-Inhibitor in dem EVOK-Modell durch Überwachen der Zellabtötung;
(ii) Testen der Empfindlichkeit gegenüber dem MEK-Inhibitor und dem SRC-Inhibitor in Zellen des EVOK-Modells durch Bestimmen eines Niveaus der MEK-Phosphorylierung (pMEK), wobei ein Niveau unter einem vorbestimmten Niveau oder einem Kontrollniveau auf eine wirksame Behandlung hinweist; und/oder
(iii) Testen der Empfindlichkeit gegenüber dem SRC-Inhibitor in Zellen des EVOK-Modells durch Bestimmen des Niveaus der ERK1/2-Phosphorylierung (pERK1/2), wobei ein Niveau unter einem vorbestimmten Niveau oder einem Kontrollniveau auf eine wirksame Behandlung hinweist.

5. Verfahren nach einem der Ansprüche 3 und 4, wobei das EVOC-Modell ein Kultivieren einer präzise geschnittenen Gewebescheibe des CRC in einem Kulturmedium umfasst, wobei die präzise geschnittene Gewebescheibe in einer stark sauerstoffhaltigen Atmosphäre mit etwa 80 % Sauerstoff gehalten wird und wobei die Kultur rotierend geschüttelt wird, um ein intermittierendes Eintauchen der Gewebescheibe in das Kulturmedium zu erleichtern.

6. Verfahren nach Anspruch 5, wobei die Kultur in einer Gewebekulturplatte erfolgt.

7. Verfahren nach einem der Ansprüche 5 oder 6, wobei das Kulturmedium DMEM/F12 umfasst.

8. Verfahren nach einem der Ansprüche 5 bis 7, wobei die Kultivierung mindestens 4 oder 5 Tage lang durchgeführt wird.

9. Verfahren nach einem der Ansprüche 5 bis 8, wobei die Scheibe in direktem Kontakt mit dem Gewebekultureinsatz steht.

10. Verfahren nach einem der Ansprüche 5 bis 9, wobei der Gewebekultureinsatz ein Titangittereinsatz ist.

11. Kombination zur Verwendung nach Anspruch 1, wobei die Krebszellen mindestens eines der folgenden Merkmale aufweisen:
(i) Empfindlichkeit gegenüber dem MEK-Inhibitor und dem SRC-Inhibitor in einem EVOK-Modell, überwacht durch Zellabtötung;
(ii) Empfindlichkeit gegenüber dem MEK-Inhibitor und dem SRC-Inhibitor in einem EVOK-Modell basierend auf einem Niveau der MEK-Phosphorylierung (pMEK), wobei ein Niveau unter einem vorbestimmten Niveau oder einem Kontrollniveau auf eine wirksame Behandlung hinweist; und/oder
(iii) Empfindlichkeit gegenüber dem SRC-Inhibitor in einem EVOK-Modell basierend auf einem Niveau der ERK1/2-Phosphorylierung (pERK1/2), wobei ein Niveau unter einem vorbestimmten Niveau oder einem Kontrollniveau auf eine wirksame Behandlung hinweist.

12. Verfahren oder Kombination zur Verwendung nach einem der Ansprüche 1 bis 10, wobei der MEK-Inhibitor aus der Gruppe ausgewählt ist, die aus Trametinib und Selumetinib besteht.

13. Verfahren oder Kombination zur Verwendung nach einem der Ansprüche 1 bis 12, wobei der SRC-Inhibitor aus der Gruppe ausgewählt ist, die aus Bosutinib, Dasatinib, Saracotinib, Ponatinib, TPX-0022 und 1-NM-PP1 besteht.

14. Verfahren oder Kombination zur Verwendung nach einem der Ansprüche 1 bis 13, ferner umfassend eine Behandlung mit Folfox oder Folfiri.

15. Verfahren oder Kombination zur Verwendung nach einem der Ansprüche 1 bis 14, wobei der CRC ausgewählt ist aus der Gruppe bestehend aus lokal fortgeschrittenem, metastasiertem CRC und einer rezidivierenden Erkrankung.

## Revendications

1. Combinaison comprenant une quantité thérapeutiquement efficace d'un inhibiteur de kinase régulée par un signal extracellulaire/mitogène (MEK) et un inhibiteur de la tyrosine kinase (SRC) non récepteur proto-oncogène SRC
pour le traitement du cancer colorectal (CRC), dans laquelle les cellules du CRC expriment une quantité de SRC p419 supérieure à un niveau prédéterminé et n'expriment pas de mutation G12 du KRAS.

2. Méthode *ex-vivo* de sélection d'un traitement contre le cancer colorectal (CRC) pour un sujet qui en a besoin, la méthode comprenant la détermination d'un niveau d'inhibiteur de la tyrosine kinase (SRC) non récepteur proto-oncogène SRC p419 et d'une mutation RAS pour exclure une mutation KRAS G12 dans les cellules cancéreuses du sujet, dans lequel un niveau de SRC p419 supérieur à un niveau prédéterminé et l'absence de mutation KRAS G12 indiquent un traitement efficace avec un inhibiteur de la kinase régulée par le signal extracellulaire/mitogène (MEK) et un inhibiteur de la SRC.

3. Méthode de la revendication 2, qui consiste en outre à tester la sensibilité à ce traitement dans un modèle de culture d'organe *ex-vivo* (EVOC).

4. Méthode de la revendication 3, comprenant l'un des éléments suivants :
(i) tester la sensibilité à l'inhibiteur de MEK et à l'inhibiteur de SRC dans le modèle EVOK en surveillant la destruction des cellules ;
(ii) tester la sensibilité à l'inhibiteur de MEK et à l'inhibiteur de SRC dans les cellules du modèle EVOK en déterminant le niveau de phosphorylation de MEK (pMEK), un niveau inférieur à un niveau prédéterminé ou à un niveau de contrôle indiquant un traitement efficace ; et/ou
(iii) tester la sensibilité à l'inhibiteur de SRC dans les cellules du modèle EVOK en déterminant le niveau de phosphorylation ERK1/2 (pERK1/2), un niveau inférieur à un niveau prédéterminé ou de contrôle étant indicatif d'un traitement efficace.

5. Méthode de l'une des revendications 3 et 4, dans laquelle le modèle EVOC comprend la culture dans un milieu de culture d'une tranche de tissu de la CRC coupée avec précision et placée sur un insert de culture de tissu, dans lequel la tranche de tissu coupée avec précision est maintenue dans une atmosphère hautement oxygénée contenant environ 80 % d'oxygène et dans lequel la culture est agitée par rotation pour faciliter la submersion intermittente de la tranche de tissu dans le milieu de culture.

6. Méthode de la revendication 5, dans laquelle ladite culture se trouve dans une plaque de culture tissulaire.

7. Méthode de l'une des revendications 5 ou 6, dans laquelle le milieu de culture comprend du DMEM/F12.

8. Méthode de l'une des revendications 5 à 7, dans laquelle la culture est effectuée pendant au moins 4 ou 5 jours.

9. Méthode de l'une des revendications 5 à 8, dans laquelle la tranche est en contact direct avec l'insert de culture tissulaire.

10. Méthode de l'une des revendications 5 à 9, dans laquelle l'insert de culture tissulaire est un insert de grille en titane.

11. Combinaison d'utilisation de la revendication 1, dans laquelle les cellules cancéreuses présentent au moins l'une des caractéristiques suivantes :
(i) la sensibilité audit inhibiteur de MEK et audit inhibiteur de SRC dans un modèle EVOK, contrôlée par la destruction des cellules ;
(ii) la sensibilité audit inhibiteur de MEK et audit inhibiteur de SRC dans un modèle EVOK basé sur le niveau de phosphorylation de MEK (pMEK), dans lequel un niveau inférieur à un niveau prédéterminé ou de contrôle indique un traitement efficace ; et/ou
(iii) la sensibilité à cet inhibiteur de SRC dans un modèle EVOK basé sur le niveau de phosphorylation de ERK1/2 (pERK1/2), où un niveau inférieur à un niveau prédéterminé ou de contrôle est indicatif d'un traitement efficace.

12. Méthode ou combinaison pour l'utilisation de l'une des revendications 1 à 10, dans laquelle l'inhibiteur de MEK est choisi dans le groupe constitué par le trametinib et le selumetinib.

13. Méthode ou combinaison pour l'utilisation de l'une des revendications 1 à 12, dans laquelle l'inhibiteur de SRC est choisi dans le groupe constitué par le bosutinib, le dasatinib, le saracotinib, le ponatinib, le TPX-0022 et le 1-NM-PP1.

14. Méthode ou combinaison pour l'utilisation de l'une des revendications 1 à 13, comprenant en outre un traitement par Folfox ou Folfiri.

15. Méthode ou combinaison pour l'utilisation de l'une des revendications 1 à 14, dans laquelle le CCR est choisi dans le groupe constitué par le CCR localement avancé, le CCR métastatique et une maladie récurrente.
